# EUROPÄISCHE PATENTSCHRIFT

(11) **EP 1 414 302 B1**
(45) Veröffentlichungstag und Bekanntmachung des Hinweises auf die Patenterteilung: **21.03.2007**
(21) Anmeldenummer: 02790165.1
(22) Anmeldetag: 16.07.2002
(51) Int. Cl.: A01N 43/90, C07D 487/04

(54) **7-AMINOTRIAZOLOPYRIMIDINE ZUR BEKÄMPFUNG VON SCHADPILZEN**
7-AMINO TRIAZOLOPYRIMIDINES FOR CONTROLLING HARMFUL FUNGI
7-AMINO-TRIAZOLOPYRIMIDINES POUR LA LUTTE CONTRE DES CHAMPIGNONS NUISIBLES

(30) Priorität: 26.07.2001 DE 10136118
(43) Veröffentlichungstag der Anmeldung: 06.05.2004
(73) Patentinhaber: BASF Aktiengesellschaft, 67056 Ludwigshafen (DE)
(72) Erfinder: TORMO I BLASCO, Jordi, 69469 Weinheim (DE); SAUTER, Hubert, 72270 Baiersbronn (DE); MÜLLER, Bernd, 67227 Frankenthal (DE); GEWEHR, Markus, 56288 Kastellaun (DE); GRAMMENOS, Wassilios, 67071 Ludwigshafen (DE); GROTE, Thomas, 67157 Wachenheim (DE); GYPSER, Andreas, 68159 Mannheim (DE); RHEINHEIMER, Joachim, 67063 Ludwigshafen (DE); ROSE, Ingo, 68159 Mannheim (DE); SCHÄFER, Peter, 67308 Ottersheim (DE); SCHIEWECK, Frank, 67098 Bad Dürkheim (DE); AMMERMANN, Eberhard, 64646 Heppenheim (DE); STRATHMANN, Siegfried, 67117 Limburgerhof (DE); LORENZ, Gisela, 67434 Neustadt (DE); STIERL, Reinhard, 67251 Freinsheim (DE); KRUMMEL, Günter, 55578 Vendersheim (DE)
(86) Internationale Anmeldenummer: PCT/EP2002/007893
(87) Internationale Veröffentlichungsnummer: WO 2003/009687

(56) Entgegenhaltungen:
- EP-A- 0 613 900
- WO-A-94/20501
- US-A- 5 612 345

## Beschreibung

Die vorliegende Erfindung betrifft 7-Aminotriazolopyrimidine der Formel I, 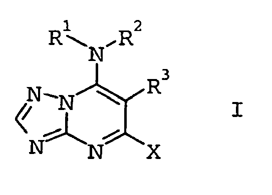 in der die Substituenten die folgenden Bedeutungen haben:
- R¹, R²: Wasserstoff, C₁-C₁₀-Alkyl, C₂-C₁₀-Alkenyl, C₂-C₁₀-Alkinyl, C₃-C₈-Cycloalkyl, Phenyl, Naphthyl; oder
5- oder 6-gliedriges Heterocyclyl enthaltend ein bis vier Stickstoffatome oder ein bis drei Stickstoffatome und ein Schwefel- oder Sauerstoffatom; oder
5- oder 6-gliedriges Heteroaryl enthaltend ein bis vier Stickstoffatome oder ein bis drei Stickstoffatome und ein Schwefel- oder Sauerstoffatom,
wobei R¹ und R², wenn ungleich Wasserstoff, unabhängig voneinander teilweise oder vollständig halogeniert sein können und/oder ein bis drei Reste aus der Gruppe R^{a}
R^{a} Cyano, Nitro, Hydroxy, C₁-C₆-Alkyl, C₁-C₆-Haloalkyl, C₃-C₆-Cycloalkyl, C₁-C₆-Alkoxy, C₁-C₆-Haloalkoxy, C₁-C₆-Alkylthio, C₁-C₆-Alkylamino, Di-C₁-C₆-alkylamino, C₂-C₆-Alkenyl, C₂-C₆-Alkenyloxy, C₂-C₆-Alkinyl, C₃-C₆-Alkinyloxy und gegebenenfalls halogeniertes Oxy-C₁-C₄-alkylenoxy, tragen können; oder
R¹ und R² können zusammen mit dem Stickstoffatom, das sie verbindet, einen 5- oder 6-gliedrigen Ring bilden, der ein bis vier Stickstoffatome oder ein bis drei Stickstoffatome und ein Schwefel- oder Sauerstoffatom enthält, und der durch ein bis drei Reste aus der Gruppe R^{a} substituiert sein kann;
- R³: C₁-C₁₀-Alkyl, C₂-C₁₀-Alkenyl, C₂-C₁₀-Alkinyl, C₃-C₈-Cycloalkyl, Phenyl-C₁-C₁₀-Alkyl,
wobei R³ unsubstituiert oder teilweise oder vollständig halogeniert sein kann und/oder ein bis drei Reste aus der Gruppe R^{a} tragen kann, oder
C₁-C₁₀-Haloalkyl, das ein bis drei Reste aus der Gruppe R^{a} tragen kann;
- X: Halogen, Cyano, C₁-C₄-Alkoxy, C₁-C₄-Haloalkyl, Phenyl oder durch R^{a} substituiertes Phenyl;
sowie deren Salze.

Zusätzlich betrifft die Erfindung Verfahren zur Herstellung der Verbindungen I, sowie Mittel und die Verwendung der Verbindungen I zur Bekämpfung von Schadpilzen.

6-Aryl-Triazolopyrimidine werden in WO 98/46608 und EP-A 550 113 offenbart. Durch aromatische Gruppen speziell substituierte 6-Benzyl-Triazolopyrimidine mit pharmazeutischer Wirkung sind aus US 5,231,094 und US 5,387,747 bekannt. EP-A 141 317 offenbart 7-Aminotriazolopyrimidine, welche in 5-Position einen Alkylrest tragen können. 6-Cycloalkyltriazolopyrimidine mit diversen Resten in 5-Position werden in EP-A 613 900 genannt. 5,7-Dihalogen-Triazolopyrimidine werden in US 5,612,345 und WO 94/20501 vorgeschlagen.

Die in WO 98/46608, EP-A 550 113, EP-A 141 317, EP-A 613 900, US 5,612,345 und WO 94/20501 beschriebenen Verbindungen sind als Pflanzenschutzmittel gegen Schadpilze geeignet.

Ihre Wirkung ist jedoch in vielen Fällen nicht zufriedenstellend. Daher lag als Aufgabe zugrunde, Verbindungen mit verbesserter Wirksamkeit zu finden.

Demgemäß wurden die 7-Aminotriazolopyrimidine der Formel I gefunden. Weiterhin wurden Zwischenprodukte und Verfahren zur Herstellung der Verbindungen I, sowie die Verwendung der Verbindungen I und diese enthaltende Mittel zur Bekämpfung von Schadpilzen gefunden.

Die Verbindungen der Formel I unterscheiden sich von den aus den oben genannten Schriften bekannten Verbindungen durch die Kombination der Substituenten X mit dem Rest R³ am Triazolopyrimidingerüst.

Verbindungen der Formel I, wobei X Halogen bedeutet, erhält man beispielsweise ausgehend von Dicarbonylverbindungen der Formel II.1, welche man mit 3-Amino-1,2,4-triazol der Formel III zu Hydroxytriazolopyrimidinen der Formel IV.1 cyclisiert:

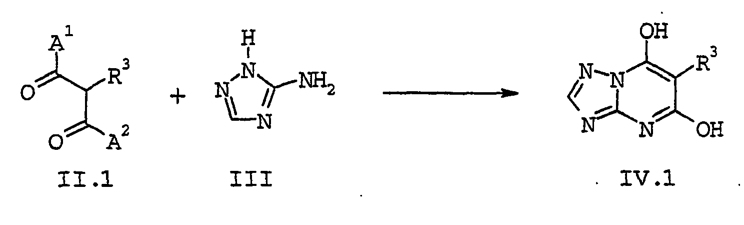

Diese Umsetzung erfolgt üblicherweise bei Temperaturen von 25 °C bis 210 °C, vorzugsweise 120 °C bis 180 °C, in Gegenwart einer Base [vgl. EP-A-770615] .

Als Basen kommen allgemein organische Basen, z.B. tertiäre Amine wie Trimethylamin, Triethylamin, Triisopropyl-ethylamin, Tributylamin und N-Methylpiperidin, Pyridin in Betracht. Besonders bevorzugt werden Triethylamin und Tributylamin.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, II.1 in einem Überschuß bezogen auf III einzusetzen.

Die für die Herstellung der Verbindungen I benötigten Ausgangsstoffe sind in der Literatur bekannt oder können gemäß der zitierten Literatur hergestellt werden [Heterocycl. 1996, pp. 1031 - 1047; Tetrahedron Lett. 1966, 24, 2661 - 2668] oder sind kommerziell zugänglich.

Anschließend setzt man die Hydroxytriazolopyrimidine der Formel IV.1 mit einem Halogenierungsmittel zu Halogentriazolopyrimidinen der Formel V.1 um:

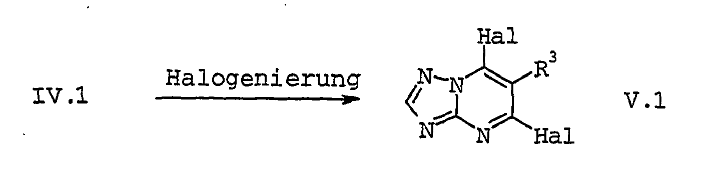

Diese Umsetzung erfolgt üblicherweise bei Temperaturen von 0 °C bis 150 °C, vorzugsweise 80 °C bis 125 °C, in einem inerten organischen Lösungsmittel oder ohne zusätzliches Lösungsmittel [vgl.EP-A-770 615].

Geeignete Halogenierungsmittel sind bevorzugt Bromierungs- oder Chlorierungsmittel, wie beispielsweise Phosphoroxybromid oder Phosphoroxychlorid, in Substanz oder in Anwesendheit eines Lösungsmittels.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, besonders bevorzugt Toluol, o-, m- und p-Xylol.

Es können auch Gemische der genannten Lösungsmittel verwendet werden.

Anschließend setzt man die Halogentriazolopyrimidine der Formel V.1 mit einem Amin der Formel VI zu 7-Aminotriazolopyrimidinen der Formel I um, in der X Halogen bedeutet:

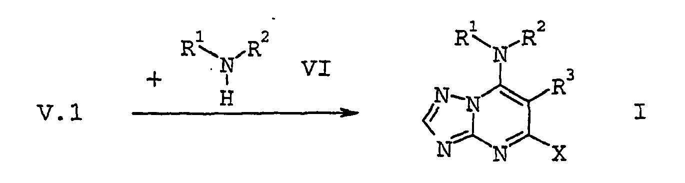

Diese Umsetzung erfolgt üblicherweise bei Temperaturen von 0 °C bis 70 °C, vorzugsweise 10 °C bis 35 °C, in einem inerten organischen Lösungsmittel in Gegenwart einer Base [vgl. EP-A-550 113].

Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat, Kaliumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium, außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Triethylamin, Kaliumcarbonat und Natriumcarbonat.

Die Basen werden im allgemeinen in katalytischen Mengen eingesetzt, sie können aber auch äquimolar, im Überschuß oder gegebenenfalls als Lösungsmittel verwendet werden. Alternativ dazu kann ein Überschuß der Verbindung VI als Base dienen.

Die Edukte werden im allgemeinen in äquimolaren Mengen miteinander umgesetzt. Es kann für die Ausbeute vorteilhaft sein, VI in einem Überschuß bezogen auf V.I einzusetzen.

Um 7-Aminotriazolopyrimidine der Formel I zu erhalten, in denen X Cyano oder C₁-C₄-Alkoxy bedeutet, werden 7-Aminotriazolo-pyrimidine der Formel I mit einer Verbindung der Formel VII umgesetzt:

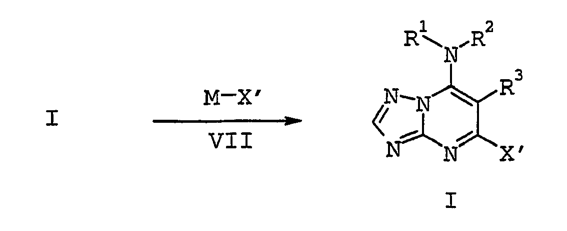

Dabei bedeutet M ein Ammonium-, Tetraalkylammonium-, Alkalimetall- oder Erdalkalimetallkation und X' Cyano oder Alkoxy.

Diese Umsetzung erfolgt üblicherweise bei Temperaturen von 0 °C bis 150 °C, vorzugsweise 20 °C bis 75 °C, in einem inerten organischen Lösungsmittel [vgl. WO 99/41255].

Geeignete Lösungsmittel sind Ether wie Diethylether, Diisopropylether, tert.-Butylmethylether, Dioxan, Anisol und Tetrahydrofuran, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt Diethylether, Tetrahydrofuran, Methanol und Dimethylformamid.

Es können auch Gemische der genannten Lösungsmittel verwendet werden.

Die Edukte werden im allgemeinen in äquimolaren Mengen miteinander umgesetzt. Es kann für die Ausbeute vorteilhaft sein, VII in einem Überschuß bezogen auf I einzusetzen.

7-Aminotriazolopyrimidine der Formel I, in der X C₁-C₄-Haloalkyl oder gegebenenfalls durch R^{a} substituiertes Phenyl bedeutet, sind erhältlich ausgehend von Dicarbonylverbindungen der Formel II.2, die man mit 3-Amino-1,2,4-triazol der Formel III zu 7-Hydroxytriazolopyrimidinen der Formel IV.2 cyclisiert :

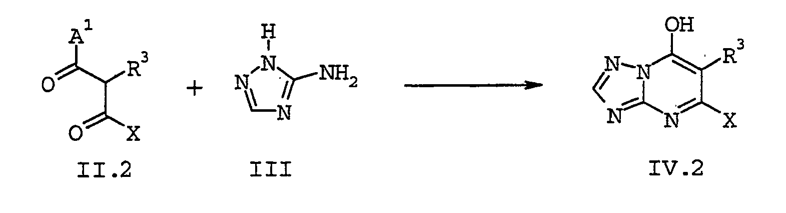

Diese Umsetzung erfolgt unter den gleichen Bedingungen wie die vorstehend beschriebene Umsetzung von II.1 zu IV.1.

Anschließend setzt man die 7-Hydroxytriazolopyrimidine der Formel IV.2 mit einem Halogenierungsmittel zu 7-Halogentriazolopyrimidinen der Formel V.2 um:

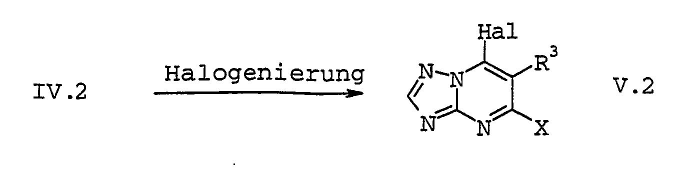

Diese Umsetzung erfolgt unter den gleichen Bedingungen wie die vorstehend beschriebene Umsetzung von IV.1 zu V.1.

Dann wird Verbindung V.2 mit einem Amin der Formel VI zu Verbindungen der Formel I, umgesetzt:

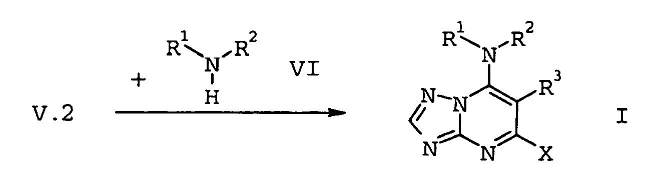

Diese Umsetzung erfolgt unter den gleichen Bedingungen wie die vorstehend beschriebene Umsetzung von V.1 zu I.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Sofern einzelne Verbindungen I nicht auf den voranstehend beschriebenen Wegen zugänglich sind, können sie durch Derivatisierung anderer Verbindungen I hergestellt werden.

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Jod;
**Alkyl:** gesättigte, geradkettige oder verzweigte Kohlenwasserstoffreste mit 1 bis 4, 6, 8 oder 10 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
**Haloalkyl:** geradkettige oder verzweigte Alkylgruppen mit 1 bis 10 Kohlenstoffatomen (wie vorstehend genannt), wobei in diesen Gruppen teilweise, beispielsweise ein- bis dreifach, oder vollständig die Wasserstoffatome durch Halogenatome wie vorstehend genannt ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
**Alkoxy:** geradkettige oder verzweigte Alkylgruppen mit 1 bis 6 Kohlenstoffatomen (wie vorstehend genannt), welche über ein Sauerstoffatom (-O-) an das Gerüst gebunden sind;
**Haloalkoxy:** geradkettige oder verzweigte Halogenalkylgruppen mit 1 bis 6 Kohlenstoffatomen (wie vorstehend genannt), welche über ein Sauerstoffatom (-O-) an das Gerüst gebunden sind;
**Alkylthio:** geradkettige oder verzweigte Alkylgruppen mit 1 bis 6 Kohlenstoffatomen (wie vorstehend genannt), welche über ein Schwefelatom (-S-) an das Gerüst gebunden sind;
**Alkylamino:** eine geradkettige oder verzweigte Alkylgruppe mit 1 bis 6 Kohlenstoffatomen (wie vorstehend genannt), welche über eine Aminogruppe (-NH-) an das Gerüst gebunden ist;
**Dialkylamino:** zwei voneinander unabhängige geradkettige oder verzweigte Alkylgruppen mit jeweils 1 bis 6 Kohlenstoffatomen (wie vorstehend genannt), welche über ein Stickstoffatom an das Gerüst gebunden sind;
**Alkenyl:** ungesättigte, geradkettige oder verzweigte Kohlenwasserstoffreste mit 2 bis 6 oder 10 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-l-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1propenyl und 1-Ethyl-2-methyl-2-propenyl;
**Alkenyloxy:** ungesättigte, geradkettige oder verzweigte Kohlenwasserstoffreste mit 3 bis 6 Kohlenstoffatomen und einer Doppelbindung in einer beliebigen, nicht zum Heteroatom benachbarten, Position (wie vorstehend genannt), welche über ein Sauerstoffatom (-O-) an das Gerüst gebunden sind;
**Alkinyl:** geradkettige oder verzweigte Kohlenwasserstoffgruppen mit 2 bis 6 oder 10 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
**Alkinyloxy:** ungesättigte, geradkettige oder verzweigte Kohlenwasserstoffreste mit 3 bis 6 Kohlenstoffatomen und einer Dreifachbindung in einer beliebigen, nicht zum Heteroatom benachbarten, Position (wie vorstehend genannt), welche über ein Sauerstoffatom (-O-) an das Gerüst gebunden sind;
**Cycloalkyl:** monocyclische, gesättigte Kohlenwasserstoffgruppen mit 3 bis 5, 6,oder 8 Kohlenstoffringgliedern, z.B. C₃-C₈-Cycloalkyl wie Cyclopropyl, Cyclobutyl, Cyclopentyl, Cyclohexyl, Cycloheptyl und Cyclooctyl;
**5- oder 6-gliedrige Heterocyclen** (Heterocyclyl) enthaltend neben Kohlenstoffringgliedern ein bis vier Stickstoffatome und/oder ein Sauerstoff- oder Schwefelatom oder ein Sauerstoff- und/oder Schwefelatom, z.B. 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl;
**5-gliedriges Heteroaryl, enthaltend** ein bis vier Stickstoffatome oder ein bis drei Stickstoffatome und ein Schwefel- oder Sauerstoffatom: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
**6-gliedriges Heteroaryl,** enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl;
**Oxyalkylenoxy:** divalente unverzweigte Ketten aus 1 bis 3 CH₂-Gruppen, wobei beide Valenzen über ein Sauerstoffatom an das Gerüst gebunden ist, z.B. OCH₂O, OCH₂CH₂O und OCH₂CH₂CH₂O;

Die Verbindungen der Formel I können auch in Form ihrer landwirtschaftlich brauchbaren Salze vorliegen, wobei es in der Regel nicht auf die Art des Salzes ankommt. Im Allgemeinen kommen die Salze derjenigen Kationen oder die Säureadditionssalze derjenigen Säuren in Betracht, deren Kationen beziehungsweise Anionen die fungizide Wirkung der Verbindungen I nicht negativ beeinträchtigen.

Es kommen als Kationen insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di(2-hydroxyeth-1-yl)ammonium, Trimethylbenzylammonium, des weiteren, Phosphoniumionen, Sufoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionssalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexaflourosilikat, Hexafluorophosphat, Benzoat sowie die Anionen von C₁-C₄-Alkansäuren, vorzugsweise Formiat, Acetat, Propionat und Butyrat.

Im Hinblick auf ihre bestimmungsgemäße Verwendung der 7-Aminotriazolopyrimidine der Formel I sind die folgenden Bedeutungen der Substituenten, und zwar jeweils für sich allein oder in Kombination, besonders bevorzugt:
Verbindungen I, in denen R¹, R² für Wasserstoff, C₁-C₁₀-Alkyl oder C₁-C₆-Haloalkyl, insbesondere für Wasserstoff, C₁-C₆-Alkyl, C₁-C₄-Haloalkyl, besonders bevorzugt für Wasserstoff, 1-Methylpropyl, Isopropyl oder 1,1,1-Trifluor-2-propyl steht, oder
R¹ und R² zusammen mit dem Stickstoffatom, das sie verbindet, einen 5-oder 6-gliedrigen Ring bilden, der ein Sauerstoffatom enthalten kann und/oder einen C₁-C₄-Alkylrest tragen kann, beispielsweise Pyrrolidin-1-yl, Pyrrol-1-yl, Pyrazol-1-yl, Imidazol-1-yl, Piperidin-1-yl, Morpholin-4-yl, wobei die genannten Reste durch ein bis drei Reste R^{a}, insbesondere C₁-C₄-Alkyl, wie beispielsweise Methyl oder Ethyl, substituiert sein können.

Daneben werden auch Verbindungen I besonders bevorzugt, in denen R¹ Wasserstoff, C₁-C₆-Alkyl und C₁-C₄-Haloalkyl und R² Wasserstoff bedeutet.

Ganz besonders werden auch Verbindungen I bevorzugt, in denen R¹ und R² Wasserstoff und R³ C₃-C₈-Cycloalkyl, bevorzugt Cyclopropyl, Cyclopentyl oder Cyclohexyl bedeuten.

Außerdem werden Verbindungen I besonders bevorzugt, in denen R³ C₁-C₈-Alkyl, insbesondere Isopropyl oder n-Octyl, C₃-C₆-Cycloalkyl, besonders bevorzugt Cyclopropyl, Cyclopentyl oder Cyclohexyl, oder CH₂-C₆H₅ bedeutet.

Insbesondere werden auch Verbindungen I bevorzugt, in denen R³ C₃-C₈-Cycloalkyl, insbesondere C₃-C₆-Cycloalkyl, besonders bevorzugt Cyclopropyl, Cyclopropylmethyl, Cyclopentyl oder Cyclohexyl, und X Cyano, C₁-C₄-Alkoxy, beispielsweise OCH₃, C₁-C₄-Haloalkyl, beispielsweise CF₃ oder ein gegebenenfalls durch R^{a} substituiertes Phenylalkyl, beispielsweise CH₂-C₆H₅ oder CH₂-O-Cl-C₆H₄, bedeutet.

Außerdem werden Verbindungen I besonders bevorzugt, in denen R³ C₃-C₈-Cycloalkyl, insbesondere C₃-C₆-Cycloalkyl, besonders bevorzugt Cyclopropyl, Cyclopentyl oder Cyclohexyl, und X Halogen, insbesondere Chlor, bedeutet.

Gleichermaßen besonders bevorzugt sind Verbindungen I, in denen X Halogen, wie Chlor oder Brom, insbesondere Chlor bedeutet.

Insbesondere sind im Hinblick auf ihre Verwendung die in den folgenden Tabellen zusammengestellten Verbindungen I bevorzugt. Die in den Tabellen für einen Substituenten genannten Gruppen stellen außerdem für sich betrachtet, unabhängig von der Kombination, in der sie genannt sind, eine besonders bevorzugte Ausgestaltung des betreffenden Substituenten dar.

### Tabelle 1

Verbindungen der Formel I, in denen R³ für CH₃ und X für Cl steht und die Kombination der Reste R¹ und R² für eine Verbindung jeweils einer Zeile der Tabelle A entspricht

### Tabelle 2

Verbindungen der Formel I, in denen R³ für CH₂-CH₃ und X für C1 steht und die Kombination der Reste R¹ und R² für eine Verbindung jeweils einer Zeile der Tabelle A entspricht

### Tabelle 3

Verbindungen der Formel I, in denen R³ für (CH₂)₃-CH₃ und X für Cl steht und die Kombination der Reste R¹ und R² für eine Verbindung jeweils einer Zeile der Tabelle A entspricht

### Tabelle 4

Verbindungen der Formel I, in denen R³ für CH₂-CH(CH₃)₂ und X für Cl steht und die Kombination der Reste R¹ und R² für eine Verbindung jeweils einer Zeile der Tabelle A entspricht

### Tabelle 5

Verbindungen der Formel I, in denen R³ für CH(CH₃)-CH₂-CH₂-CH₃ und X für Cl steht und die Kombination der Reste R¹ und R² für eine Verbindung jeweils einer Zeile der Tabelle A entspricht

### Tabelle 6

Verbindungen der Formel I, in denen R³ für C(CH₃)₃ und X für Cl steht und die Kombination der Reste R¹ und R² für eine Verbindung jeweils einer Zeile der Tabelle A entspricht

### Tabelle 7

Verbindungen der Formel I, in denen R³ für (CH₂)₇-CH₃ und X für Cl steht und die Kombination der Reste R¹ und R² für eine Verbindung jeweils einer Zeile der Tabelle A entspricht

### Tabelle 8

Verbindungen der Formel I, in denen R³ für CH(CH₃)₂ und X für C1 steht und die Kombination der Reste R¹ und R² für eine Verbindung jeweils einer Zeile der Tabelle A entspricht

### Tabelle 9

Verbindungen der Formel I, in denen R³ für Cyclopentyl und X für C1 steht und die Kombination der Reste R¹ und R² für eine Verbindung jeweils einer Zeile der Tabelle A entspricht

### Tabelle 10

Verbindungen der Formel I, in denen R³ für Cyclohexyl und X für Cl steht und die Kombination der Reste R¹ und R² für eine Verbindung jeweils einer Zeile der Tabelle A entspricht

### Tabelle 11

Verbindungen der Formel I, in denen R³ für CH₂-C₆H₅ und X für Cl steht und die Kombination der Reste R¹ und R² für eine Verbindung jeweils einer Zeile der Tabelle A entspricht

### Tabelle 12

Verbindungen der Formel I, in denen R³ für CH₂-o-Cl-C₆H₄ und X für Cl steht und die Kombination der Reste R¹ und R² für eine Verbindung jeweils einer Zeile der Tabelle A entspricht

### Tabelle 13

Verbindungen der Formel I, in denen R³ für (CH₂)₂-CH₃ und X für Cl steht und die Kombination der Reste R¹ und R² für eine Verbindung jeweils einer Zeile der Tabelle A entspricht

### Tabelle 14

Verbindungen der Formel I, in denen R³ für CH₂-CH=CH₂ und X für C1 steht und die Kombination der Reste R¹ und R² für eine Verbindung jeweils einer Zeile der Tabelle A entspricht

### Tabelle 15

Verbindungen der Formel I, in denen R³ für Cyclopropyl-methyl und X für Cl steht und die Kombination der Reste R¹ und R² für eine Verbindung jeweils einer Zeile der Tabelle A entspricht

### Tabelle 16

Verbindungen der Formel I, in denen R³ für CH₂-CH₂-CN und X für Cl steht und die Kombination der Reste R¹ und R² für eine Verbindung jeweils einer Zeile der Tabelle A entspricht

### Tabelle 17

Verbindungen der Formel I, in denen R³ für CH₂-CF₃ und X für Cl steht und die Kombination der Reste R¹ und R² für eine Verbindung jeweils einer Zeile der Tabelle A entspricht

### Tabelle 18

Verbindungen der Formel I, in denen R³ für CH(CH₃)-CF₃ und X für C1 steht und die Kombination der Reste R¹ und R² für eine Verbindung jeweils einer Zeile der Tabelle A entspricht

### Tabelle 19

Verbindungen der Formel I, in denen R³ für CH(CF₃)₂ und X für Cl steht und die Kombination der Reste R¹ und R² für eine Verbindung jeweils einer Zeile der Tabelle A entspricht

### Tabelle 20

Verbindungen der Formel I, in denen R³ für CH₃ und X für CF₃ steht und die Kombination der Reste R¹ und R² für eine Verbindung jeweils einer Zeile der Tabelle A entspricht

### Tabelle 21

Verbindungen der Formel I, in denen R³ für CH₂-CH₃ und X für CF₃ steht und die Kombination der Reste R¹ und R² für eine Verbindung jeweils einer Zeile der Tabelle A entspricht

### Tabelle 22

Verbindungen der Formel I, in denen R³ für (CH₂)₃-CH₃ und X für CF₃ steht und die Kombination der Reste R¹ und R² für eine Verbindung jeweils einer Zeile der Tabelle A entspricht

### Tabelle 23

Verbindungen der Formel I, in denen R³ für CH₂-CH(CH₃)₂ und X für CF₃ steht und die Kombination der Reste R¹ und R² für eine Verbindung jeweils einer Zeile der Tabelle A entspricht

### Tabelle 24

Verbindungen der Formel I, in denen R³ für CH(CH₃)-CH₂-CH₂-CH₃ und X für CF₃ steht und die Kombination der Reste R¹ und R² für eine Verbindung jeweils einer Zeile der Tabelle A entspricht

### Tabelle 25

Verbindungen der Formel I, in denen R³ für CH(CH₃)₃ und X für CF₃ steht und die Kombination der Reste R¹ und R² für eine Verbindung jeweils einer zeile der Tabelle A entspricht

### Tabelle 26

Verbindungen der Formel I, in denen R³ für (CH₂)₇-CH₃ und X für CF₃ steht und die Kombination der Reste R¹ und R² für eine Verbindung jeweils einer Zeile der Tabelle A entspricht

### Tabelle 27

Verbindungen der Formel I, in denen R³ für CH(CH₃)₂ und X für CF₃ steht und die Kombination der Reste R¹ und R² für eine Verbindung jeweils einer Zeile der Tabelle A entspricht

### Tabelle 28

Verbindungen der Formel I, in denen R³ für Cyclopentyl und X für CF₃ steht und die Kombination der Reste R¹ und R² für eine Verbindung jeweils einer Zeile der Tabelle A entspricht

### Tabelle 29

Verbindungen der Formel I, in denen R³ für Cyclohexyl und X für CF₃ steht und die Kombination der Reste R¹ und R² für eine Verbindung jeweils einer Zeile der Tabelle A entspricht

### Tabelle 30

Verbindungen der Formel I, in denen R³ für CH₂-C₆H₅ und X für CF₃ steht und die Kombination der Reste R¹ und R² für eine Verbindung jeweils einer Zeile der Tabelle A entspricht

### Tabelle 31

Verbindungen der Formel I, in denen R³ für CH₂-p-Cl-C₆H₄ und X für CF₃ steht und die Kombination der Reste R¹ und R² für eine Verbindung.jeweils einer Zeile der Tabelle A entspricht

### Tabelle 32

Verbindungen der Formel I, in denen R³ für (CH₂)₂-CH₃ und X für CF₃ steht und die Kombination der Reste R¹ und R² für eine Verbindung jeweils einer Zeile der Tabelle A entspricht

### Tabelle 33

Verbindungen der Formel I, in denen R³ für CH₂-CH=CH₂ und X für CF₃ steht und die Kombination der Reste R¹ und R² für eine Verbindung jeweils einer Zeile der Tabelle A entspricht

### Tabelle 34

Verbindungen der Formel I, in denen R³ für Cyclopropyl-methyl und X für CF₃ steht und die Kombination der Reste R¹ und R² für eine Verbindung jeweils einer Zeile der Tabelle A entspricht

### Tabelle 35

Verbindungen der Formel I, in denen R³ für CH₂-CH₂-CN und X für CF₃ steht und die Kombination der Reste R¹ und R² für eine Verbindung jeweils einer Zeile der Tabelle A entspricht

### Tabelle 36

Verbindungen der Formel I, in denen R³ für CH₂-CF₃ und X für CF₃ steht und die Kombination der Reste R¹ und R² für eine Verbindung jeweils einer Zeile der Tabelle A entspricht

### Tabelle 37

Verbindungen der Formel I, in denen R³ für CH(CH₃)-CF₃ und X für CF₃ steht und die Kombination der Reste R¹ und R² für eine Verbindung jeweils einer Zeile der Tabelle A entspricht

### Tabelle 38

Verbindungen der Formel I, in denen R³ für CH(CF₃)₂ und X für CF₃ steht und die Kombination der Reste R¹ und R² für eine Verbindung jeweils einer Zeile der Tabelle A entspricht

### Tabelle 39

Verbindungen der Formel I, in denen R³ für CH₃ und X für Phenyl steht und die Kombination der Reste R¹ und R² für eine Verbindung jeweils einer Zeile der Tabelle A entspricht

### Tabelle 40

Verbindungen der Formel I, in denen R³ für CH₂-CH₃ und X für Phenyl steht und die Kombination der Reste R¹ und R² für eine Verbindung jeweils einer Zeile der Tabelle A entspricht

### Tabelle 41

Verbindungen der Formel I, in denen R³ für (CH₂)₃-CH₃ und X für Phenyl steht und die Kombination der Reste R¹ und R² für eine Verbindung jeweils einer Zeile der Tabelle A entspricht

### Tabelle 42

Verbindungen der Formel I, in denen R³ für CH₂-CH(CH₃)₂ und X für Phenyl steht und die Kombination der Reste R¹ und R² für eine Verbindung jeweils einer Zeile der Tabelle A entspricht

### Tabelle 43

Verbindungen der Formel I, in denen R³ für CH(CH₃)-CH₂-CH₂-CH₃ und X für Phenyl steht und die Kombination der Reste R¹ und R² für eine Verbindung jeweils einer Zeile der Tabelle A entspricht

### Tabelle 44

Verbindungen der Formel I, in denen R³ für CH(CH₃)₃ und X für Phenyl steht und die Kombination der Reste R¹ und R² für eine Verbindung jeweils einer Zeile der Tabelle A entspricht

### Tabelle 45

Verbindungen der Formel I, in denen R³ für (CH₂)₇-CH₃ und X für Phenyl steht und die Kombination der Reste R¹ und R² für eine Verbindung jeweils einer Zeile der Tabelle A entspricht

### Tabelle 46

Verbindungen der Formel I, in denen R³ für CH(CH₃)₂ und X für Phenyl steht und die Kombination der Reste R¹ und R² für eine Verbindung jeweils einer Zeile der Tabelle A entspricht

### Tabelle 47

Verbindungen der Formel I, in denen R³ für Cyclopentyl und X für Phenyl steht und die Kombination der Reste R¹ und R² für eine Verbindung jeweils einer Zeile der Tabelle A entspricht

### Tabelle 48

Verbindungen der Formel I, in denen R³ für Cyclohexyl und X für Phenyl steht und die Kombination der Reste R¹ und R² für eine Verbindung jeweils einer Zeile der Tabelle A entspricht

### Tabelle 49

Verbindungen der Formel I, in denen R³ für CH₂-C₆H₅ und X für Phenyl steht und die Kombination der Reste R¹ und R² für eine Verbindung jeweils einer Zeile der Tabelle A entspricht

### Tabelle 50

Verbindungen der Formel I, in denen R³ für CH₂-p-Cl-C₆H₄ und X für Phenyl steht und die Kombination der Reste R¹ und R² für eine Verbindung jeweils einer Zeile der Tabelle A entspricht

### Tabelle 51

Verbindungen der Formel I, in denen R³ für (CH₂)₂-CH₃. und X für Phenyl steht und die Kombination der Reste R¹ und R² für eine Verbindung jeweils einer Zeile der Tabelle A entspricht

### Tabelle 52

Verbindungen der Formel I, in denen R³ für CH₂-CH=CH₂ und X für Phenyl steht und die Kombination der Reste R¹ und R² für eine Verbindung jeweils einer Zeile der Tabelle A entspricht

### Tabelle 53

Verbindungen der Formel I, in denen R³ für Cyclopropyl-methyl und X für Phenyl steht und die Kombination der Reste R¹ und R² für eine Verbindung jeweils einer Zeile der Tabelle A entspricht

### Tabelle 54

Verbindungen der Formel I, in denen R³ für -CH₂-CH₂-CN und X für Phenyl steht und die Kombination der Reste R¹ und R² für eine Verbindung jeweils einer Zeile der Tabelle A entspricht

### Tabelle 55

Verbindungen der Formel I, in denen R³ für CH₂-CF₃ und X für Phenyl steht und die Kombination der Reste R¹ und R² für eine Verbindung jeweils einer Zeile der Tabelle A entspricht

### Tabelle 56

Verbindungen der Formel I, in denen R³ für CH(CH₃)-CF₃ und X für Phenyl steht und die Kombination der Reste R¹ und R² für eine Verbindung jeweils einer Zeile der Tabelle A entspricht

### Tabelle 57

Verbindungen der Formel I, in denen R³ für CH(CF₃)₂ und X für Phenyl steht und die Kombination der Reste R¹ und R² für eine Verbindung jeweils einer Zeile der Tabelle A entspricht

### Tabelle 58

Verbindungen der Formel I, in denen R³ für CH₃ und X für CN steht und die Kombination der Reste R¹ und R² für eine Verbindung jeweils einer Zeile der Tabelle A entspricht

### Tabelle 59

Verbindungen der Formel I, in denen R³ für CH₂-CH₃ und X für CN steht und die Kombination der Reste R¹ und R² für eine Verbindung jeweils einer Zeile der Tabelle A entspricht

### Tabelle 60

Verbindungen der Formel I, in denen R³ für (CH₂)₃-CH₃ und X für CN steht und die Kombination der Reste R¹ und R² für eine Verbindung jeweils einer Zeile der Tabelle A entspricht

### Tabelle 61

Verbindungen der Formel I, in denen R³ für CH₂-CH(CH₃)₂ und X für CN steht und die Kombination der Reste R¹ und R² für eine Verbindung jeweils einer Zeile der Tabelle A entspricht.

### Tabelle 62

Verbindungen der Formel I, in denen R³ für CH(CH₃)-CH₂-CH₂-CH₃ und X für CN steht und die Kombination der Reste R¹ und R² für eine Verbindung jeweils einer Zeile der Tabelle A entspricht

### Tabelle 63

Verbindungen der Formel I, in denen R³ für CH(CH₃)₃ und X für CN steht und die Kombination der Reste R¹ und R² für eine Verbindung jeweils einer Zeile der Tabelle A entspricht

### Tabelle 64

Verbindungen der Formel I, in denen R³ für (CH₂)₇-CH₃ und X für CN steht und die Kombination der Reste R¹ und R² für eine Verbindung jeweils einer Zeile der Tabelle A entspricht

### Tabelle 65

Verbindungen der Formel I, in denen R³ für CH(CH₃)₂ und X für CN steht und die Kombination der Reste R¹ und R² für eine Verbindung jeweils einer Zeile der Tabelle A entspricht

### Tabelle 66

Verbindungen der Formel I, in denen R³ für Cyclopentyl und X für CN steht und die Kombination der Reste R¹ und R² für eine Verbindung jeweils einer Zeile der Tabelle A entspricht

### Tabelle 67

Verbindungen der Formel I, in denen R³ für Cyclohexyl und X für CN steht und die Kombination der Reste R¹ und R² für eine Verbindung jeweils einer Zeile der Tabelle A entspricht

### Tabelle 68

Verbindungen der Formel I, in denen R³ für CH₂-C₆H₅ und X für CN steht und die Kombination der Reste R¹ und R² für eine Verbindung jeweils einer Zeile der Tabelle A entspricht

### Tabelle 69

Verbindungen der Formel I, in denen R³ für CH₂-p-Cl-C₆H₄ und X für CN steht und die Kombination der Reste R¹ und R² für eine Verbindung jeweils einer Zeile der Tabelle A entspricht.

### Tabelle 70

Verbindungen der Formel I, in denen R³ für (CH₂)₂-CH₃ und X für CN steht und die Kombination der Reste R¹ und R² für eine Verbindung jeweils einer Zeile der Tabelle A entspricht

### Tabelle 71

Verbindungen der Formel I, in denen R³ für CH₂-CH=CH₂ und X für CN steht und die Kombination der Reste R¹ und R² für eine Verbindung jeweils einer Zeile der Tabelle A entspricht

### Tabelle 72

Verbindungen der Formel I, in denen R³ für Cyclopropyl-methyl und X für CN steht und die Kombination der Reste R¹ und R² für eine Verbindung jeweils einer Zeile der Tabelle A entspricht

### Tabelle 73

Verbindungen der Formel I, in denen R³ für CH₂-CH₂-CN und X für CN steht und die Kombination der Reste R¹ und R² für eine Verbindung jeweils einer Zeile der Tabelle A entspricht

### Tabelle 74

Verbindungen der Formel I, in denen R³ für CH₂-CF₃ und X für CN steht und die Kombination der Reste R¹ und R² für eine Verbindung jeweils einer Zeile der Tabelle A entspricht

### Tabelle 75

Verbindungen der Formel I, in denen R³ für CH(CH₃)-CF₃ und X für CN steht und die Kombination der Reste R¹ und R² für eine Verbindung jeweils einer Zeile der Tabelle A entspricht

### Tabelle 76

Verbindungen der Formel I, in denen R³ für CH(CF₃)₂ und X für CN steht und die Kombination der Reste R¹ und R² für eine Verbindung jeweils einer Zeile der Tabelle A entspricht

### Tabelle 77

Verbindungen der Formel I, in denen R³ für CH₃ und X für OCH₃ steht und die Kombination der Reste R¹ und R² für eine Verbindung jeweils einer Zeile der Tabelle A entspricht

### Tabelle 78

Verbindungen der Formel I, in denen R³ für CH₂-CH₃ und X für OCH₃ steht und die Kombination der Reste R¹ und R² für eine Verbindung jeweils einer Zeile der Tabelle A entspricht

### Tabelle 79

Verbindungen der Formel I, in denen R³ für (CH₂)₃-CH₃ und X für OCH₃ steht und die Kombination der Reste R¹ und R² für eine Verbindung jeweils einer Zeile der Tabelle A entspricht

### Tabelle 80

Verbindungen der Formel I, in denen R³ für CH₂-CH(CH₃)₂ und X für OCH₃ steht und die Kombination der Reste R¹ und R² für eine Verbindung jeweils einer Zeile der Tabelle A entspricht

### Tabelle 81

Verbindungen der Formel I, in denen R³ für CH(CH₃)-CH₂-CH₂-CH₃ und X für OCH₃ steht und die Kombination der Reste R¹ und R² für eine Verbindung jeweils einer Zeile der Tabelle A entspricht

### Tabelle 82

Verbindungen der Formel I, in denen R³ für CH(CH₃)₃ und X für OCH₃ steht und die Kombination der Reste R¹ und R² für eine Verbindung jeweils einer Zeile der Tabelle A entspricht

### Tabelle 83

Verbindungen der Formel I, in denen R³ für (CH₂)₇-CH₃ und X für OCH₃ steht und die Kombination der Reste R¹ und R² für eine Verbindung jeweils einer Zeile der Tabelle A entspricht

### Tabelle 84

Verbindungen der Formel I, in denen R³ für CH(CH₃)₂ und X für OCH₃ steht und die Kombination der Reste R¹ und R² für eine Verbindung jeweils einer Zeile der Tabelle A entspricht

### Tabelle 85

Verbindungen der Formel I, in denen R³ für Cyclopentyl und X für OCH₃ steht und die Kombination der Reste R¹ und R² für eine Verbindung jeweils einer Zeile der Tabelle A entspricht

### Tabelle 86

Verbindungen der Formel I, in denen R³ für Cyclohexyl und X für OCH₃ steht und die Kombination der Reste R¹ und R² für eine Verbindung jeweils einer Zeile der Tabelle A entspricht

### Tabelle 87

Verbindungen der Formel I, in denen R³ für CH₂-C₆H₅ und X für OCH₃ steht und die Kombination der Reste R¹ und R² für eine Verbindung jeweils einer Zeile der Tabelle A entspricht

### Tabelle 88

Verbindungen der Formel I, in denen R³ für CH₂-p-Cl-C₆H₄ und X für OCH₃ steht und die Kombination der Reste R¹ und R² für eine Verbindung jeweils einer Zeile der Tabelle A entspricht

### Tabelle 89

Verbindungen der Formel I, in denen R³ für (CH₂)₂-CH₃ und X für OCH₃ steht und die Kombination der Reste R¹ und R² für eine Verbindung jeweils einer Zeile der Tabelle A entspricht

### Tabelle 90

Verbindungen der Formel I, in denen R³ für CH₂-CH=CH₂ und X für OCH₃ steht und die Kombination der Reste R¹ und R² für eine Verbindung jeweils einer Zeile der Tabelle A entspricht

### Tabelle 91

Verbindungen der Formel I, in denen R³ für Cyclopropyl-methyl und X für OCH₃ steht und die Kombination der Reste R¹ und R² für eine Verbindung jeweils einer Zeile der Tabelle A entspricht

### Tabelle 92

Verbindungen der Formel I, in denen R³ für CH₂-CH₂-CN und X für OCH₃ steht und die Kombination der Reste R¹ und R² für eine Verbindung jeweils einer Zeile der Tabelle A entspricht

### Tabelle 93

Verbindungen der Formel I, in denen R³ für CH₂-CF₃ und X für OCH₃ steht und die Kombination der Reste R¹ und R² für eine Verbindung jeweils einer Zeile der Tabelle A entspricht

### Tabelle 94

Verbindungen der Formel I, in denen R³ für CH(CH₃)-CF₃ und X für OCH₃ steht und die Kombination der Reste R¹ und R² für eine Verbindung jeweils einer Zeile der Tabelle A entsprich

### Tabelle 95

Verbindungen der Formel I, in denen R³ für CH(CF₃)₂ und X für OCH₃ steht und die Kombination der Reste R¹ und R² für eine Verbindung jeweils einer Zeile der Tabelle A entspricht

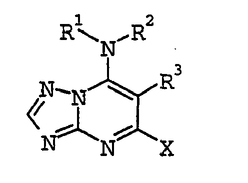

**Tabelle A**

| **No.** | **R¹** | **R²** |
|---|---|---|
| A-1 | H | H |
| A-2 | CH₂CH₃ | H |
| A-3 | CH₂CH₃ | CH₃ |
| A-4 | CH₂CH₃ | CH₂CH₃ |
| A-5 | CH₂CF₃ | H |
| A-6 | CH₂CF₃ | CH₃ |
| A-7 | CH₂CF₃ | CH₂CH₃ |
| A-8 | CH₂CCl₃ | H |
| A-9 | CH₂CCl₃ | CH₃ |
| A-10 | CH₂CCl₃ | CH₂CH₃ |
| A-11 | CH₂CH₂CH₃ | H |
| A-12 | CH₂CH₂CH₃ | CH₃ |
| A-13 | CH₂CH₂CH₃ | CH₂CH₃ |
| A-14 | CH₂CH₂CH₃ | CH₂CH₂CH₃ |
| A-15 | CH(CH₃)₂ | H |
| A-16 | CH(CH₃)₂ | CH₃ |
| A-17 | CH(CH₃)₂ | CH₂CH₃ |
| A-18 | (*R*/*S*) CH(CH₃)-CH₂CH₃ | H |
| A-19 | (*R*/*S*) CH(CH₃)-CH₂CH₃ | CH₃ |
| A-20 | (*R*/*S*) CH(CH₃)-CH₂CH₃ | CH₂CH₃ |
| A-21 | (*R*) CH(CH₃)-CH₂CH₃ | H |
| A-22 | (*R*) CH(CH₃)-CH₂CH₃ | CH₃ |
| A-23 | (*R*) CH(CH₃)-CH₂CH₃ | CH₂CH₃ |
| A-24 | (*S*) CH(CH₃)-CH₂CH₃ | H |
| A-25 | (*S*) CH(CH₃)-CH₂CH₃ | CH₃ |
| A-26 | (*S*) CH(CH₃)-CH₂CH₃ | CH₂CH₃ |
| A-27 | (*R*/*S*) CH(CH₃)-CH(CH₃)₂ | H |
| A-28 | (*R*/*S*) CH(CH₃)-CH(CH₃)₂ | CH₃ |
| A-29 | (*R*/*S*) CH(CH₃)-CH(CH₃)₂ | CH₂CH₃ |
| A-30 | (*R*) CH(CH₃)-CH(CH₃)₂ | H |
| A-31 | (*R*) CH(CH₃)-CH(CH₃)₂ | CH₃ |
| A-32 | (*R*) CH(CH₃)-CH(CH₃)₂ | CH₂CH₃ |
| A-33 | (*S*) CH(CH₃)-CH(CH₃)₂ | H |
| A-34 | (*S*) CH(CH₃)-CH(CH₃)₂ | CH₃ |
| A-35 | (*S*) CH(CH₃)-CH(CH₃)₂ | CH₂CH₃ |
| A-36 | (*R*/*S*) CH(CH₃)-C(CH₃)₃ | H |
| A-37 | (*R*/*S*) CH(CH₃)-C(CH₃)₃ | CH₃ |
| A-38 | (*R*/*S*) CH(CH₃)-C(CH₃)₃ | CH₂CH₃ |
| A-39 | (*R*) CH(CH₃)-C(CH₃)₃ | H |
| A-40 | (*R*) CH(CH₃)-C(CH₃)₃ | CH₃ |
| A-41 | (*R*) CH(CH₃)-C(CH₃)₃ | CH₂CH₃ |
| A-42 | (*S*) CH(CH₃)-C(CH₃)₃ | H |
| A-43 | (*S*) CH(CH₃)-C(CH₃)₃ | CH₃ |
| A-44 | (*S*) CH(CH₃)-C(CH₃)₃ | CH₂CH₃ |
| A-45 | (*R*/*S*) CH(CH₃)-CF₃ | H |
| A-46 | (*R*/*S*) CH(CH₃)-CF₃ | CH₃ |
| A-47 | (*R*/*S*) CH(CH₃)-CF₃ | CH₂CH₃ |
| A-48 | (*R*) CH(CH₃)-CF₃ | H |
| A-49 | (*R*) CH(CH₃)-CF₃ | CH₃ |
| A-50 | (*R*) CH(CH₃)-CF₃ | CH₂CH₃ |
| A-51 | (*S*) CH(CH₃)-CF₃ | H |
| A-52 | (*S*) CH(CH₃)-CF₃ | CH₃ |
| A-53 | (*S*) CH(CH₃)-CF₃ | CH₂CH₃ |
| A-54 | (*R*/*S*) CH(CH₃)-CCl₃ | H |
| A-55 | (*R*/*S*) CH(CH₃)-CCl₃ | CH₃ |
| A-56 | (*R*/*S*) CH(CH₃)-CCl₃ | CH₂CH₃ |
| A-57 | (*R*) CH(CH₃)-CCl₃ | H |
| A-58 | (*R*) CH(CH₃)-CCl₃ | CH₃ |
| A-59 | (*R*) CH(CH₃)-CCl₃ | CH₂CH₃ |
| A-60 | (*S*) CH(CH₃)-CCl₃ | H |
| A-61 | (*S*) CH(CH₃)-CCl₃ | CH₃ |
| A-62 | (*S*) CH(CH₃)-CCl₃ | CH₂CH₃ |
| A-63 | CH₂C(CH₃)=CH₂ | H |
| A-64 | CH₂C(CH₃)=CH₂ | CH₃ |
| A-65 | CH₂C(CH₃)=CH₂ | CH₂CH₃ |
| A-66 | cyclopentyl | H |
| A-67 | cyclopentyl | CH₃ |
| A-68 | cyclopentyl | CH₂CH₃ |
| A-69 | - (CH₂)₂CH (CH₃) (CH₂)₂- | |

Die besonders bevorzugten Ausführungsformen der Zwischenprodukte in Bezug auf die Variablen entsprechen denen der Reste R¹, R², R^{a}, R³ und X der Formel I.

Die Verbindungen I eignen sich als Fungizide. Sie zeichnen sich durch eine hervorragende Wirksamkeit gegen ein breites Spektrum von pflanzenpathogenen Pilzen, insbesondere aus der Klasse der *Ascomyceten, Deuteromyceten, Phycomyceten* und *Basidiomyceten,* aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
· Alternaria-Arten, Podosphaera-Arten, Sclerotinia-Arten, Physalospora canker an Gemüse und Obst,
· Botrytis cinerea (Grauschimmel) an Erdbeeren, Gemüse, Zierpflanzen und Reben,
· Corynespora cassiicola an Gurken,
· Colletotrichum-Arten an Obst und Gemüse,
· Diplocarpon rosae an Rosen,
· Elsinoe fawcetti und Diaporthe citri an Citrus-Früchten,
· Sphaerotheca-Arten an Kürbisgewächsen, Erdbeeren und Rosen,
· Cercospora-Arten an Erdnüssen, Zuckerrüben und Auberginen,
· Erysiphe cichoracearum an Kürbisgewächsen,
· Leveillula taurica an Paprika, Tomaten und Auberginen,
· Mycosphaerella-Arten an Äpfeln und japanischer Aprikose, · Phyllactinia kakicola, Gloesporium kaki, an japanischer Aprikose,
· Gymnosporangium yamadae, Leptothyrium pomi, Podosphaera leucotricha und Gloedes pomigena an Äpfeln,
· Cladosporium carpophilum an Birnen und japanischer Aprikose,
·Phomopsis-Arten an Birnen,
· Phytophthora-Arten an Citrusfrüchten, Kartoffeln, Zwiebeln, insbesondere Phytophthora infestans an Kartoffeln und Tomaten,
· Blumeria graminis (echter Mehltau) an Getreide,
· Fusarium- und Verticillium-Arten an verschiedenen Pflanzen,
· Glomerella cingulata an Tee,
· Drechslera- und Bipolaris- Arten an Getreide und Reis,
· Mycosphaerella-Arten an Bananen und Erdnüssen,
· Plasmopara viticola an Reben,
· Personospora-Arten an Zwiebeln, Spinat und Chrysantemen,
· Phaeoisariopsis vitis und Sphaceloma ampelina an Grapefruits,
· Pseudocercosporella herpotrichoides an Weizen und Gerste,
· Pseudoperonospora-Arten an Hopfen und Gurken,
· Puccinia-Arten und Typhula-Arten an Getreide und Rasen,
· Pyricularia oryzae an Reis,
· Rhizoctonia-Arten an Baumwolle, Reis und Rasen,
· Stagonospora nodorum und Septoria tritici an Weizen,
· Uncinula necator an Reben,
· Ustilago-Arten an Getreide und Zuckerrohr, sowie
· Venturia-Arten (Schorf) an Äpfeln und Birnen.

Die Verbindungen I eignen sich außerdem zur Bekämpfung von Schadpilzen wie Paecilomyces variotii im Materialschutz (z.B. Holz, Papier, Dispersionen für den Anstrich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B.Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.
II. 30 Gew.-Teile einer erfindungsgemäßen Verbindung werden mit einer Mischung aus 92 Gew.-Teilen pulverförmigem Kieselsäuregel und8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.-%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5Gew.-Teilen des Anlagerungsproduktes von 40 Mol Ethylenoxid an 1 Mol Ricinusöl besteht (Wirkstoffgehalt 16 Gew.-%).
V. 80 Gew.-Teile einer erfindungsgemäßen Verbindung werden mit 3 Gew.-Teilen des Natriumsalzes der Diisobutylnaphthalinalpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).
VI. Man vermischt 90 Gew.-Teile einer erfindungsgemäßen Verbindung mit 10 Gew.-Teilen N-Methyl-a-pyrrolidon und erhält eine Lösung, die zur Anwendung in Form kleinster Tropfen geeignet ist (Wirkstoffgehalt 90 Gew.-%).
VII. 20 Gew.-Teile einer erfindungsgemäßen Verbindung werden in einer Mischung gelöst, die aus 40 Gew.-Teilen Cyclohexanon, 30Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
VIII. 20 Gew.-Teile einer erfindungsgemäßen Verbindung werden mit 3 Gew.-Teilen des Natriumsalzes der Diisobutylnaphthalin-a-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Ö1 bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoffohne Zusätze auszubringen.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemä-Ben Verbindungen gemeinsam angewendet werden können, soll die Kombinationsmöglichkeiten erläutern, nicht aber einschränken:
· Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;
· Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
· heterocyclische Substanzen, wie 2-Heptadecyl-2-imidazolin-acetat, 2-Chlor-N-(4'-chlor-biphenyl-2-yl)-nicotinamid, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid,
   N-Dichlorofluoromethylthio-N',N' -dimethyl-N-phenyl-schwefelsäure-diamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethylfuran-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-1-(2,2,2-trichlorethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethyl-morpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, (2RS,3RS)-1-[3-(2-Chlorphenyl)-2-(4-fluorphenyl)-oxiran-2-yl-methyl]-1H-1,2,4-triazol, a-(2-Chlorphenyl)-a-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
· Strobilurine wie Methyl-E-methoxyimino-[a-(o-tolyloxy)-o-to-lyl]acetat, Methyl-E-2-{2-[6-(2-cyanophenoxy)-pyrimidin-4-yloxy]-phenyl}-3-methoxyacrylat, Methyl-E-methoxyimino-[a-(2-phenoxyphenyl)]-acetamid, Methyl-E-methoxyimino-[a-(2,5-dimethylphenoxy)-o-tolyl]-acetamid, Methyl-E-2-{2-[2-trifluormethylpyridyl-6-]oxymethyl]-phenyl}3-methoxyacrylat, (E,E)-Methoximino-{2-[1-(3-trifluormethylphenyl)-ethylidenaminooxymethyl]-phenyl}-essigsäuremethylester, Methyl-N-(2-{[1-(4-chlorphenyl)-1H-pyrazol-3-yl]oxymethyl}-phenyl)N-methoxy-carbamat,
· Anilinopyrimidine wie N-(4,6-Dimethylpyrimidin-2-yl)-anilin, N-[4-Methyl-6-(1-propinyl)-pyrimidin-2-yl]-anilin, N-[4-Methyl-6-cyclopropyl-pyrimidin-2-yl]-anilin,
Phenylpyrrole wie 4-(2,2-Difluor-1,3-benzodioxol-4-yl)-pyrrol-3-carbonitril,
· Zimtsäureamide wie 3-(4-Chlorphenyl)-3-(3,4-dimethoxyphenyl)-acrylsäuremorpholid, 3-(4-Fluorphenyl)-3-(3,4-dimethoxyphenyl)-acrylsäuremorpholid,
· sowie verschiedene Fungizide, wie Dodecylguanidinacetat, 1-(3-Brom-6-methoxy-2-methyl-phenyl)-1-(2,3,4-trimethoxy-6-methyl-phenyl)-methanon, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methyl-ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-(3,5-Dichlorphenyl)-5-methyl-5-methoxymethyl-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbo-nyl)-2-methoximino]-acetamid, 1-[2-(2,4-Dichlorphenyl)-pen-tyl]-1H-1,2,4-triazol, 2,4-Difluor-a-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol, 5-Chlor-2-cyano-4-p-tolyl-imidazol-1-sulfonsäuredimethylamid, 3,5-Dichlor-N-(3-chlor-1-ethyl-1-methyl-2-oxo-propyl)-4-methyl-benzamid.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle mit physikalischen Angaben aufgeführt.

### Beispiel 1 Herstellung von 5,7-Dihydroxy-6-isopropyl-[1,2,4]-triazolo-[1,5-α]-pyrimidin

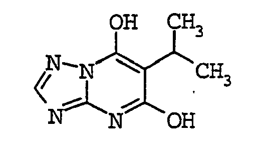

Eine Mischung von 14 g (0,17 mol) 3-Amino-1,2,4-triazol, 34,3 g (0,17 mol) Diethyl-2-isopropylmalonat und 50 ml Tributylamin wurden 6 h bei 180°C gerührt. Dann wurde die Reaktionsmischung auf 70 °C abgekühlt, eine wäßrige Lösung von Natriumhydroxid (21g/200ml Wasser) zugegeben und die Mischung 30 min gerührt. Die organische Phase wurde abgetrennt und die wäßrige Phase wurde mit Diethylether extrahiert. Anschließend wurde die wäßrige Phase mit konz. Salzsäure angesäuert und der sich bildende Niederschlag durch Filtration gesammelt. Nach Trocknen erhielt man 27 g (0,14 mol) der Titelverbindung.

### Beispiel 2 Herstellung von 5,7-Dichlor-6-isopropyl-[1,2,4]-triazolo-[1,5-α]-pyrimidin

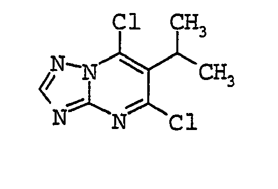

Eine Mischung von 25 g (0,13 mol) 5,7-Dichlor-6-isopropyl-[1,2,4]-triazolo[1,5-α]-pyrimidin (vgl. Bsp. 1) und 50 ml Phosphoroxychlorid wurden 8 h refluxiert. Dann wurde das Phosphoroxychlorid teilweise abdestilliert und der Rückstand in eine Mischung aus Methylenchlorid und Wasser gegossen. Die organische Phase wurde abgetrennt, getrocknet und filtriert. Das Filtrat wurde vom Lösungsmittel befreit. Man erhielt 16 g (0,07 mol) der Titelverbindung (Schmelzpunkt 119 °C).

### Beispiel 3 Herstellungvon 5-Chlor-6-isopropyl-7-cyclopentylamino-[1,2,4]-triazolo-[1,5-α]-pyrimidin

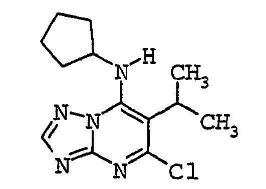

Eine Mischung von 0,13 g (1,5 mmol) Cyclopentylamin, 0,15 g (1,5 mmol) Triethylamin in 10 ml Methylenchlorid wurde unter Rühren zu einer Mischung von 0,34 g (1,5 mmol)5,7-Dichlor-6-isopropyl-[1,2,4]-triazolo-[1,5-α]-pyrimidin (vgl. Bsp. 2) in 20 ml Methylenchlorid gegeben. Die Reaktionsmischung wurde bei Raumtemperatur 16 h gerührt und anschließend mit 5%iger Salzsäure gewaschen. Die organische Phase wurde abgetrennt, über Natriumsulfat getrocknet und filtriert. Das Filtrat wurde vom Lösungsmittel befreit und der Rückstand chromatographisch gereinigt. Man erhielt 0,32 g (1,14 mmol) der Titelverbindung (Schmelzpunkt 139°C) .

### Beispiel 4 Herstellung von 7-Hydroxy-6-propyl-5-trifluormethyl-[1,2,4]-triazolo[1,5-α]-pyrimidin

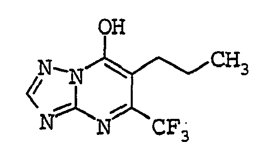

Eine Mischung von 14 g (0,17 mol) 3-Amino-1,2,4-triazol, 38,4 g (0,17 mol) 3-Oxo-2-propyl-4,4,4-trifluorobutanoat und 50 ml Tributylamin wurden 6 h bei 180 °C gerührt. Die Aufarbeitung erfolgte analog zu Bsp. 1. Nach Trocknen erhielt man 33 g (0,13 mol) der Titelverbindung.

### Beispiel 5 Herstellung von 7-Chlor-6-propyl-5-trifluormethyl-[1,2,4]-triazolo-[1,5-α]-pyrimidin

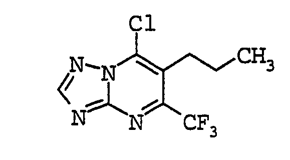

Eine Mischung von 25 g (0,10 mol) 5,7-Dichlor-6-isopropyl-[1,2,4]-triazolo[1,5-α]-pyrimidin (vgl. Bsp. 4) und 50 ml Phosphoroxychlorid wurden 8 h refluxiert. Die Aufarbeitung erfolgte analog zu Bsp. 2. Man erhielt 23 g (0,086 mol) der Titelverbindung (Schmelzpunkt 63 °C).

### Beispiel 6 Herstellung von 7-Cyclopentylamino-6-propyl-5-trifluormethyl- [1,2,4]-triazolo-[1,5-α] -pyrimidin

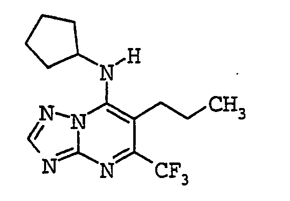

Eine Mischung von 0,13 g (1,5 mmol) Cyclopentylamin, 0, 15 g (1,5 mmol) Triethylamin in 10 ml Methylenchlorid wurde unter Rühren zu einer Mischung von 0,40 g (1,5 mmol) 7-Chlor-6-propyl-5-trifluormethyl-[1,2,4]-triazolo-[1,5-a]-pyrimidin (vgl. Bsp. 5) in 20 ml Methylenchlorid gegeben. Die Reaktionsmischung wurde bei Raumtemperatur 16 h gerührt, die Aufarbeitung erfolgt analog zu Bsp. 3. Man erhielt 0,39 g (1,24 mmol) der Titelverbindung (Schmelzpunkt 179 °C).

### Beispiel 7 Herstellung von 7-Hydroxy-6-octyl-5-phenyl-[1,2,4]-triazolo-[1,5-α]-pyrimidin

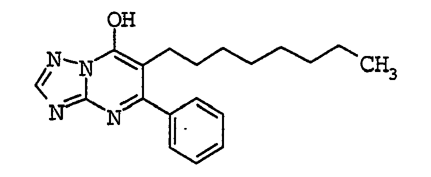

Eine Mischung von 14,0 g (0,17 mol) 3-Amino-1,2,4-triazol, 51,7 g (0,17 mol) 3-Oxo-2-octyl-4-phenylbutanoat und 3 g p-Toluolsulfonsäure wurden 6 h refluxiert. Die Aufarbeitung erfolgte analog zu Bsp. 1. Nach Trocknen erhielt man 37 g (0,11 mol) der Titelverbindung.

### Beispiel 8 Herstellung von 7-Chlor-6-octyl-5-phenyl-[1,2,4]-triazolo-[1,5-α]-pyrimidin

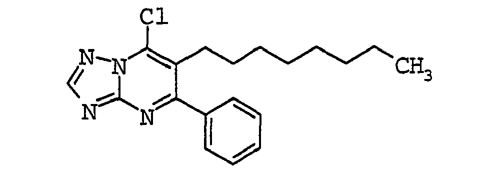

Eine Mischung von 17 g (0,05 mol) 7-Hydroxy-6-octyl-5-phenyl-[1,2,4]-triazolo-[1,5-a]-pyrimidin (vgl. Bsp. 7)und 50 ml Phosphoroxychlorid wurden 8 h refluxiert. Die Aufarbeitung erfolgte analog zu Bsp. 2. Man erhielt 16 g (0,046 mol) der Titelverbindung.

### Beispiel 9 Herstellung von 7-Cyclopentylamino-6-octyl-5-phenyl-[1,2,4]-triazolo-[1,5-α]-pyrimidin

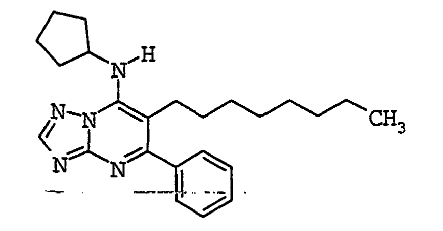

Eine Mischung von 0,13 g (1,5 mmol) Cyclopentylamin, 0,15 g (1,5 mmol) Triethylamin in 10 ml Methylenchlorid wurde unter Rühren zu einer Mischung von 0,52 g (1,5 mmol) 7-Chloro-6-octyl-5-phenyl-[1,2,4]-triazolo-[1,5-a]-pyrimidin (vgl. Bsp. 8) in 20 ml Methylenchlorid gegeben. Die Reaktionsmischung wurde bei Raumtemperatur 16 h gerührt, die Aufarbeitung erfolgt analog zu Bsp. 3. Man erhielt 0,52 g (1,3 mmol) der Titelverbindung (Schmelzpunkt 81 °C).

### Beispiel 10 Herstellung von 5-Cyano-6-octyl-7-diethylamino-[1,2,4]-triazolo-[1,5-α]-pyrimidin [I-167]

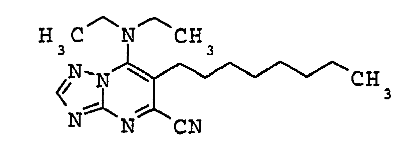

Eine Mischung von 0,1 mol der Verbindung I-48 und 0,25 mol Tetraethylammoniumcyanid in 750 ml Dimethylformamid wurde etwa 16 Std. bei 20 bis 25 °C gerührt. Nach Zusatz von Wasser und Methyl-tert.Butylether wurden die Phasen getrennt. Die organische Phase wurde nach Waschen mit Wasser und Trocknen vom Lösungsmittel befreit. Aus dem Rückstand erhielt man nach Chromatographie an Kieselgel 8,33 g der Titelverbindung.
¹H-NMR: δ in ppm: 8,5 (s); 3,65 (q); 2,9 (m); 1,7 (m); 1,3 (m); 1,2 (t); 0,9 (t).

### Beispiel 11 Herstellung von 5-Methoxy-6-octyl-7-diethylamino-[1,2,4]-triazolo-[1,5-α]-pyrimidin [I-168]

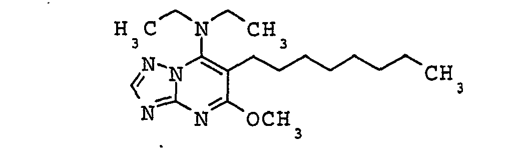

Eine Lösung von 65 mmol der Verbindung I-48 in 400 ml wasserfr. Methanol wurde bei 20 bis 25°C mit 71,5 mmol einer 30 %igen Natriummethanolat-Lösung versetzt, dann etwa 16 Std. bei 20 bis 25°C gerührt. Nach Abdestillieren des Lösungsmittels wurde der Rückstand in Dichlormethan aufgenommen. Diese Lösung wurde mit Wasser gewaschen, dann getrocknet und vom Lösungsmittel befreit. Nach Chromatographie an Kieselgel wurden 7,5 g der Titelverbindung erhalten.
¹H-NMR: δ in ppm: 8,18 (s); 4,09 (s); 3,41 (q); 2,65 (m); 1,55 (m) ; 1,3 (m); 1,1 (t); 0,9 (t).

**Tabelle 1**

| **Nr.** | **R¹** | **R²** | **R³** | **X** | **phys. Daten (m.p.[°C]; IR[cm⁻¹]; ¹H-NMR δ [ppm])** |
|---|---|---|---|---|---|
| I-1 | CH(CH₃)₂ | H | CH₃ | Cl | 169 |
| I-2 | -(CH₂)₂CH(CH₃) (CH₂)₂- | | CH₃ | Cl | 125 |
| I-3 | Cyclopentyl | H | CH₃ | Cl | 172 |
| I-4 | CH₂-CH₃ | CH₂-CH₃ | CH₃ | Cl | 96 |
| I-5 | CH(CH₃)-CF₃ | H | CH₃ | Cl | 209 |
| I-6 | CH₂-CF₃ | H | CH₃ | Cl | 133 |
| I-7 | CH₂-C(CH₃)=CH₂ | CH₂-CH₃ | CH₂-CH₃ | Cl | 55 |
| I-8 | CH(CH₃)₂ | | CH₂-CH₃ | Cl | 152 |
| I-9 | -(CH₂)₂CH(CH₃)(CH₂)₂- | | CH₂-CH₃ | C1 | 1543, 1521, 1451 |
| I-10 | Cyclopentyl | H | CH₂-CH₃ | C1 | 136 |
| I-11 | CH₂-CH₃ | CH₂-CH₃ | CH₂-CH₃ | Cl | 1596, 1511, 1464 |
| I-12 | (CH₂)₂-CH₃ | (CH₂)₂-CH₃ | CH₂-CH₃ | Cl | 1595, 1511, 1456 |
| I-13 | CH-(CH₃)₂ | CH₃ | CH₂-CH₃ | Cl | 1593, 1513, 1097 |
| I-14 | (*R*/*S*) CH (CH₃)-CH₂-CH₃ | H | CH₂-CH₃ | Cl | 145 |
| I-15 | (*R*) CH(CH₃)-CH₂-CH₃ | H | CH₂-CH₃ | Cl | 140 |
| I-16 | (*S*) CH(CH₃)-CH₂-CH₃ | H | CH₂-CH₃ | Cl | 140 |
| I-17 | (*R*/*S*) CH(CH₃)-CH(CH₃)₂ | H | CH₂-CH₃ | Cl | 119 |
| I-18 | (*R*) CH(CH₃)-CH(CH₃)₂ | H | CH₂-CH₃ | Cl | 102 |
| I-19 | (*S*) CH(CH₃)-CH(CH₃)₂ | H | CH₂-CH₃ | Cl | 102 |
| I-20 | (*R*/*S*) CH(CH₃)-C(CH₃)₃ | H | CH₂-CH₃ | Cl | 116 |
| I-21 | (*R*) CH(CH₃)-C(CH₃)₃ | H | CH₂-CH₃ | Cl | 1613, 1555, 1464 |
| I-22 | (*S*) CH(CH₃)-C(CH₃)₃ | H | CH₂-CH₃ | Cl | 1612, 1554, 1464 |
| I-23 | *(R*/*S)* CH(CH₃)-CF₃ | H | CH₂-CH₃ | Cl | 169 |
| I-24 | (*R*) CH (CH₃) -CF₃ | H | CH₂-CH₃ | Cl | 140 |
| I-25 | (*S*) CH (CH₃) -CF₃ | H | CH₂-CH₃ | Cl | 140 |
| I-26 | H | H | CH₂-CH₃ | Cl | 263 |
| I-27 | -(CH₂)₂CH(CH₃)(CH₂)₂- | | (CH₂)₃-CH₃ | Cl | 91 |
| I-28 | (*R*/*S*) CH(CH₃)-CF₃ | H | (CH₂)₃-CH₃ | Cl | 125 |
| I-29 | (*R*) CH(CH₃)-CF₃ | H | (CH₂)₃-CH₃ | Cl | 121 |
| I-30 | (*S*) CH(CH₃)-CF₃ | H | (CH₂)₃-CH₃ | Cl | 121 |
| I-31 | CH₂-CF₃ | H | (CH₂)₃-CH₃ | Cl | 156 |
| I-32 | CH₂-C(CH₃)=CH₂ | H | CH₂-CH(CH₃)₂ | Cl | 180 |
| I-33 | CH (CH₃) ₂ | H | CH₂-CH(CH₃)₂ | Cl | 127 |
| I-34 | cyclopentyl | . H | CH₂-CH(CH₃)₂ | Cl | 56 |
| I-35 | CH₂CH₃ | CH₂CH₃ | CH₂-CH(CH₃)₂ | Cl | 163 |
| I-36 | CH(CH₃)-CH₂-CH₃ | H | CH₂-CH(CH₃)₂ | Cl | 159 |
| I-37 | CH(CH₃)₂ | H | CH(CH₃)-CH₂-CH₂-CH₃ | Cl | 180 |
| I-38 | cyclopentyl | H | CH(CH₃)-CH₂-CH₂-CH₃ | Cl | 127 |
| I-39 | CH₂CH₃ | CH₂CH₃ | CH(CH₃)-CH₂-CH₂-CH₃ | Cl | 56 |
| I-40 | (*R*/*S*) CH(CH₃)-CF₃ | H | CH(CH₃)-CH₂-CH₂-CH₃ | Cl | 163 |
| I-41 | CH₂-CF₃ | H | CH(CH₃)-CH₂-CH₂-CH₃ | Cl | 159 |
| I-42 | CH(CH₃)₂ | H | C (CH₃) ₃ | Cl | 136 |
| I-43 | -(CH₂)₂CH(CH₃)(CH₂)₂- | | C (CH₃) ₃ | Cl | 140 |
| I-44 | CH₂-C(CH₃)=CH₂ | CH₂-CH₃ | (CH₂)₇-CH₃ | Cl | 2927, 1597, 1508, 1462 |
| I-45 | CH(CH₃)₂ | H | (CH₂)₇-CH₃ | Cl | 2926, 1613, 1553, 1464 |
| I-46 | -(CH₂)₂CH(CH₃)(CH₂)₂- | | (CH₂)₇-CH₃ | Cl | 2925, 1594, 1520, 1192 |
| I-47 | Cyclopentyl | H | (CH₂)₇-CH₃ | Cl | 2927, 1612, 1554, 1059 |
| I-48 | CH₂-CH₃ | CH₂-CH₃ | (CH₂)₇-CH₃ | Cl | 2927, 1598, 1511, 1466 |
| I-49 | (CH₂)₂-CH₃ | (CH₂)₂-CH₃ | (CH₂)₇-CH₃ | Cl | 2927, 1597, 1561, 1457 |
| I-50 | CH-(CH₃)₂ | CH₃ | (CH₂)₇-CH₃ | Cl | 2926, 1595, 1514, 1467 |
| I-51 | (*R*/*S*) CH(CH₃)-CH(CH₃)₂ | H | (CH₂)₇-CH₃ | Cl | 2926, 1613, 1553, 1464 |
| I-52 | (*R*) CH(CH₃)-CH(CH₃)₂ | H | (CH₂)₇-CH₃ | Cl | 2926, 1612, 1553, 1464 |
| I-53 | (*S*) CH(CH₃)-CH(CH₃)₂ | H | (CH₂)₇-CH₃ | Cl | 2926, 1612, 1552, 1463 |
| I-54 | (*R*/*S*) CH(CH₃)-C(CH₃)₃ | H | (CH₂)₇-CH₃ | Cl | 2926, 1613, 1555, 1464 |
| I-55 | (*R*) CH(CH₃)-C(CH₃)₃ | H | (CH₂)7-CH₃ | Cl | 2926, 1613, 1556, 1467 |
| I-56 | (*S*) CH(CH₃)-C(CH₃)₃ | H | (CH₂)₇-CH₃ | Cl | 2925, 1612, 1556, 1466 |
| I-57 | (*R*/*S*) CH(CH₃)-CF₃ | H | (CH₂)₇-CH₃ | Cl | 1619, 1533, 1146 |
| I-58 | (*R*) CH(CH₃)-CF₃ | H | (CH₂)₇-CH₃ | Cl | 1620, 1542, 1146 |
| I-59 | (*S*) CH(CH₃)-CF₃ | H | (CH₂)₇-CH₃ | Cl | 1619, 1541, 1146 |
| I-60 | CH₂-C(CH₃)=CH₂ | CH₂-CH₃ | CH(CH₃)₂ | Cl | 71 |
| I-61 | CH(CH₃)₂ | H | CH(CH₃)₂ | Cl | 180 |
| I-62 | CH₂-CH₃ | CH₂-CH₃ | CH(CH₃)₂ | Cl | 91 |
| I-63 | (CH₂)₂-CH₃ | (CH₂)₂-CH₃ | CH(CH₃)₂ | Cl | 1592, 1506, 1454 |
| I-64 | CH- (CH₃)₂ | CH₃ | CH (CH₃)₂ | Cl | 85 |
| I-65 | *(R*/*S)* CH(CH₃)-CH₂-CH₃ | H | CH(CH₃)₂ | Cl | 1616, 1544, 1463 |
| I-66 | (*R*) CH (CH₃) -CH₂-CH₃ | H | CH(CH₃)₂ | Cl | 160 |
| I-67 | (*S*) CH(CH₃)-CH₂-CH₃ | H | CH(CH₃)₂ | Cl | 160 |
| I-68 | (*R*/*S*) CH(CH₃)-CH(CH₃)₂ | H | CH(CH₃)₂ | Cl | 134 |
| I-69 | (*R*) CH(CH₃)-CH(CH₃)₂ | H | CH(CH₃)₂ | Cl | 120 |
| I-70 | (*S*) CH(CH₃)-CH(CH₃)₂ | H | CH(CH₃)₂ | Cl | 120 |
| I-71 | (*R*/*S*) CH(CH₃)-C(CH₃)₃ | H | CH(CH₃)₂ | Cl | 2964, 1611, 1551 |
| I-72 | (*R*) CH(CH₃)-C(CH₃)₃ | H | CH(CH₃)₂ | Cl | 64 |
| I-73 | (*S*) CH(CH₃)-C(CH₃)₃ | H | CH(CH₃)₂ | Cl | 64 |
| I-74 | (*R*/*S*) CH(CH₃)-CF₃ | H | CH(CH₃)₂ | Cl | 1616, 1527, 1147 |
| I-75 | (*R*) CH(CH₃)-CF₃ | H | CH (CH₃)₂ | Cl | 70 |
| I-76 | (*S*) CH(CH₃)-CF₃ | H | CH(CH₃)₂ | Cl | 70 |
| I-77 | H | H | CH(CH₃)₂ | Cl | 271 |
| I-78 | CH₂-C(CH₃)=CH₂ | CH₂-CH₃ | Cyclopentyl | Cl | 66 |
| I-79 | CH(CH₃)₂ | H | Cyclopentyl | Cl | 136 |
| I-80 | CH₂-CH₃ | CH₂-CH₃ | Cyclopentyl | Cl | 78 |
| I-81 | (CH₂)₂-CH₃ | (CH₂)₂-CH₃ | Cyclopentyl | Cl | 87 |
| I-82 | CH₂-C(CH₃)=CH₂ | CH₂-CH₃ | Cyclohexyl | Cl | 136 |
| I-83 | CH(CH₃)₂ | H | Cyclohexyl | Cl | 156 |
| I-84 | -(CH₂)₂CH(CH₃)(CH₂)₂- | | Cyclohexyl | Cl | 151 |
| I-85 | Cyclopentyl | H | Cyclohexyl | Cl | 158 |
| I-86 | CH₂-CH₃ | CH₂-CH₃ | Cyclohexyl | Cl | 103 |
| I-87 | (CH₂)₂-CH₃ | (CH₂)₂-CH₃ | Cyclohexyl | Cl | 139 |
| I-88 | CH-(CH₃)₂ | CH₃ | Cyclohexyl | Cl | 134 |
| I-89 | (*R*/*S*) CH(CH₃)-CH₂-CH₃ | H | Cyclohexyl | Cl | 155 |
| I-90 | (*R*) CH(CH₃)-CH₂-CH₃ | H | Cyclohexyl | Cl | 155 |
| I-91 | (*S*) CH (CH₃) -CH₂-CH₃ | H | Cyclohexyl | Cl | 155 |
| I-92 | *(R*/*S)* CH(CH₃)-CH(CH₃)₂ | H | Cyclohexyl | Cl | 114 |
| I-93 | (*R*) CH(CH₃)-CH(CH₃)₂ | H | Cyclohexyl | Cl | 110 |
| I-94 | (*S*) CH(CH₃)-CH(CH₃)₂ | H | Cyclohexyl | Cl | 110 |
| I-95 | (*R*/*S*) CH(CH₃)-C(CH₃)₃ | H | Cyclohexyl | Cl | 134 |
| I-96 | (*R*) CH(CH₃)-C(CH₃)₃ | H | Cyclohexyl | Cl | 116 |
| I-97 | (*S*) CH(CH₃)-C(CH₃)₃ | H | Cyclohexyl | Cl | 116 |
| I-98 | (*R*/*S*) CH(CH₃)-CF₃ | H | Cyclohexyl | Cl | 160 |
| I-99 | (*R*) CH(CH₃)-CF₃ | H | Cyclohexyl | Cl | 130 |
| I-100 | (*S*) CH(CH₃)-CF₃ | H | Cyclohexyl | Cl | 130 |
| I-101 | CH₂-CF₃ | H | Cyclohexyl | Cl | 167 |
| I-102 | -(CH₂)₂CH(CH₃)(CH₂)₂- | | CH₂-C₆H₅ | Cl | 144 |
| I-103 | CH₂-C(CH₃)=CH₂ | CH₂-CH₃ | CH₂-(2-Cl-C₆H₄) | Cl | 114 |
| I-104 | -(CH₂)₂CH(CH₃)(CH₂)₂- | | CH₂- (2-Cl-C₆H₄) | Cl | 164 |
| I-105 | CH₂-C(CH₃)=CH₂ | CH₂-CH₃ | CH₂-CH=CH₂ | Cl | 55 |
| I-106 | -(CH₂)₂CH(CH₃)(CH₂)₂- | | CH₂-CH=CH₂ | Cl | 37 |
| I-107 | Cyclopentyl | H | CH₂-CH=CH₂ | Cl | 43 |
| I-108 | (*R*/*S*) CH(CH₃)-CF₃ | H | Cyclopropyl-methyl | Cl | 150 |
| I-109 | CH₂-CF₃ | H | Cyclopropyl-methyl | Cl | 144 |
| I-110 | CH(CH₃)₂ | H | CH₂-CH₂-CN | Cl | 211 |
| I-111 | CH₂-C(CH₃)=CH₂ | CH₂-CH₃ | CH₂-CF₃ | Cl | 84 |
| I-112 | CH(CH₃)₂ | H | CH₂-CF₃ | Cl | 151 |
| I-113 | Cyclopentyl | H | CH₂-CF₃ | Cl | 163 |
| I-114 | CH₂-CH₃ | CH₂-CH₃ | CH₂-CF₃ | Cl | 103 |
| I-115 | (CH₂)₂-CH₃ | (CH₂)₂-CH₃ | CH₂-CF₃ | Cl | 107 |
| I-116 | CH-(CH₃)₂ | CH₃ | CH₂-CF₃ | Cl | 88 |
| I-117 | (*R*/*S*) CH(CH₃)-CH₂-CH₃ | H | CH₂-CF₃ | Cl | 131 |
| I-118 | (*R*) CH(CH₃)-CH₂-CH₃ | H | CH₂-CF₃ | Cl | 126 |
| I-119 | (*S*) CH(CH₃)-CH₂-CH₃ | H | CH₂-CF₃ | Cl | 126 |
| I-120 | (*R*/*S*) CH(CH₃)-CH(CH₃)₂ | H | CH₂-CF₃ | Cl | 114 |
| I-121 | (*R*) CH(CH₃)-CH(CH₃)₂ | H | CH₂-CF₃ | Cl | 112 |
| I-122 | (*S*) CH(CH₃)-CH(CH₃)₂ | H | CH₂-CF₃ | Cl | 112 |
| I-123 | (*R*/*S*) CH(CH₃)-C(CH₃)₃ | H | CH₂-CF₃ | Cl | 110 |
| I-124 | (*R*) CH(CH₃)-C(CH₃)₃ | H | CH₂-CF₃ | Cl | 105 |
| I-125 | (*S*) CH(CH₃)-C(CH₃) | H | CH₂-CF₃ | Cl | 105 |
| I-126 | (*R*/*S*) CH(CH₃)-CF₃ | H | CH₂-CF₃ | Cl | 179 |
| I-127 | (*R*) CH(CH₃)-CF₃ | H | CH₂-CF₃ | Cl | 125 |
| I-128 | (*S*) CH(CH₃)-CF₃ | H | CH₂-CF₃ | Cl | 125 |
| I-129 | H | H | CH₂-CF₃ | Cl | 243 |
| I-130 | CH(CH₃)₂ | H | (CH₂)₇-CH₃ | CF₃ | 91 |
| I-131 | -(CH₂)₂CH(CH₃)(CH₂)₂- | | (CH₂)₇-CH₃ | CF₃ | 64 |
| I-132 | Cyclopentyl | H | (CH₂)₇-CH₃ | CF₃ | 84 |
| I-133 | H | H | (CH₂)₇-CH₃ | CF₃ | 177 |
| I-134 | CH(CH₃)₂ | H | (CH₂)₂-CH₃ | CF₃ | 162 |
| I-135 | -(CH₂)₂CH(CH₃) (CH₂)₂- | | (CH₂)₂-CH₃ | CF₃ | 108 |
| I-136 | (*R*) CH(CH₃)-C(CH₃)₃ | H | (CH₂)₂-CH₃ | CF₃ | 101 |
| I-137 | (*S*) CH(CH₃)-C(CH₃)₃ | H | (CH₂)₂-CH₃ | CF₃ | 101 |
| I-138 | H | H | (CH₂)₂-CH₃ | CF₃ | 241 |
| I-139 | CH(CH₃)₂ | H | (CH₂)₇-CH₃ | C₆H₅ | 83 |
| I-140 | -(CH₂)₂CH(CH₃)(CH₂)₂- | | (CH₂)₇-CH₃ | C₆H₅ | 63 |
| I-141 | H | H | (CH₂)₇-CH₃ | C₆H₅ | 163 |
| I-142 | -(CH₂)₂CH(CH₃)(CH₂)₂- | | Cyclopentyl | Cl | 2960, 1610, 1550, 1241 |
| I-143 | Cyclopentyl | H | Cyclopentyl | Cl | 154 |
| I-144 | CH-(CH₃)₂ | CH₃ | Cyclopentyl | Cl | 2958, 1610, 1548, 1239 |
| I-145 | (*R*/*S*) CH(CH₃)-CH₂-CH₃ | H | Cyclopentyl | Cl | 143 |
| I-146 | (*S*) CH(CH₃)-CH₂-CH₃ | H | Cyclopentyl | Cl | 137 |
| I-147 | (*R*) CH(CH₃)-CH₂-CH₃ | H | Cyclopentyl | Cl | 137 |
| I-148 | (*R*/*S*) CH(CH₃)-CH(CH₃)₂ | H | Cyclopentyl | Cl | 124 |
| I-149 | (*S*) CH(CH₃)-CH(CH₃)₂ | H | Cyclopentyl | Cl | 110 |
| I-150 | (*R*) CH(CH₃)-CH(CH₃)₂ | H | Cyclopentyl | Cl | 110 |
| I-151 | (*R*/*S*) CH(CH₃)-C(CH₃)₃ | H | Cyclopentyl | Cl | 113 |
| I-152 | (*S*) CH(CH₃)-C(CH₃)₃ | H | Cyclopentyl | Cl | 2962, 1610, 1550, 1241 |
| I-153 | (*R*) CH(CH₃)-C(CH₃)₃ | H | Cyclopentyl | Cl | 2960, 1610, 1549, 1241 |
| I-154 | (*R*/*S*) CH(CH₃)-CF₃ | H | Cyclopentyl | C1 | 129 |
| I-155 | (*S*₎ CH(CH₃)-CF₃ | H | Cyclopentyl | Cl | 135 |
| I-156 | (*R*) CH(CH₃)-CF₃ | H | Cyclopentyl | Cl | 135 |
| I-157 | H | H | CH(CH₃)-(CH₂)₅-CH₃ | CF₃ | 129 |
| I-158 | H | H | (CH₂)₃-CH(CH₃)₂ | CF₃ | 213 |
| 1-159 | H | H | (CH₂)₆-CH₃ | CF₃ | 180 |
| I-160 | H | H | (CH₂)₅-CH₃ | CF₃ | 208 |
| I-161 | H | H | CH(CH₂CH₃)-(CH₂)₃-CH₃ | CF₃ | 162 |
| I-162 | H | H | CH(CH₂CH₂CH₃)₂ | CF₃ | 164 |
| I-163 | H | H | CH(CH₃)-CH2)₃-CH₃ | CF₃ | 148 |
| 1-164 | H | H | (CH₂)₇-CH₃ | Cl | 277 |
| I-165 | H | H | Cyclopentyl | Cl | 8,4(s); 8,2(m); 3,45(m); 1,95(m); 8,2(m); 3,45(m); |
| I-166 | H | H | Cyclohexyl | Cl | 8,45(s); 8,2(m); 3,1(m); 2,1(m); 1,8(m); 1,55(m); 1, 4 (m) |
| I-167 | CH₂-CH₃ | CH₂-CH₃ | (CH2)₇-CH₃ | CN | s. Beispiel 10 |
| I-168 | CH₂-CH₃ | CH₂-CH₃ | (CH₂)₇-CH₃ | OCH₃ | s. Beispiel 11 |

### Beispiele für die Wirkung gegen Schadpilze

Die fungizide Wirkung der Verbindungen der allgemeinen Formel I ließ sich durch die folgenden Versuche zeigen:

Die Wirkstoffe wurden getrennt oder gemeinsam als 10%ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Wettol® EM (nichtionischer Emulgator auf der Basis von ethoxyliertem Ricinusöl) aufbereitet und entsprechend der gewünschten Konzentration mit Wasser verdünnt.

### Anwendungsbeispiel 1 - Wirksamkeit gegen Botrytis cinerea an Paprikablättern

Paprikasämlinge der Sorte "Neusiedler Ideal Elite" wurden, nachdem sich 4 - 5 Blätter gut entwickelt hatten, mit einer wäßrigen Wirkstoffaufbereitung, die aus einer Stammlösung aus 10 % Wirkstoff, 85 % Cyclohexanon und 5 % Emulgiermittel angesetzt wurde, bis zur Tropfnässe besprüht. Am nächsten Tag wurden die behandelten Pflanzen mit einer Sporensuspension von *Botrytis cinerea,* die 1,7 x 10⁶ Sporen/ml in einer 2 %igen wäßrigen Biomalzlösung enthielt, inokuliert. Anschließend wurden die Versuchspflanzen in eine Klimakammer mit 22 bis 24 °C und hoher Luftfeuchtigkeit gestellt. Nach 5 Tagen konnte das Ausmaß des Pilzbefall auf den Blättern visuell in % ermittelt werden.

In diesem Test zeigten die mit 250 ppm der Wirkstoffe I-10, 1-61, 1-65, I-66, 1-68, 1-69, 1-76, I-78, 1-84, 1-100, 1-101, 1-146 und 1-153 bis I-155 behandelten Pflanzen keinen oder bis zu 15 % Befall, während die unbehandelten Pflanzen zu 90 % befallen waren.

### Anwendungsbeispiel 2 - Wirksamkeit gegen den falschen Mehltau an Reben (Plasmopara viticola)

Blätter von Topfreben der Sorte "Müller-Thurgau" wurden mit wäßriger Wirkstoffaufbereitung, die mit einer Stammlösung aus 10 % Wirkstoff, 85 % Cyclohexanon und 5 % Emulgiermittel angesetzt wurde, bis zur Tropfnässe besprüht. Am folgenden Tag wurden die Blätter mit einer wäßrigen Zoosporenaufschwemmung von *Plasmopara viticola* inokuliert. Danach wurden die Reben zunächst für 48 Stunden in einer wasserdampfgesättigten Kammer bei 24 °C und anschließend für 5 Tage im Gewächshaus bei Temperaturen zwischen 20 und 30 °C aufgestellt. Nach dieser Zeit wurden die Pflanzen zur Beschleunigung des Sporangienträgerausbruchs abermals für 16 Stunden in eine feuchte Kammer gestellt. Dann wurde das Ausmaß der Befallsentwicklung auf den Blattunterseiten visuell ermittelt.

In diesem Test zeigten die mit 250 ppm der Wirkstoffe I-8 bis I-10, I-19, I-25, I-27, I-49, I-60 bis I-62, I-69, I-84, I-101, I-113, I-133, I-146, und I-153 bis I-155 behandelten Pflanzen keinen oder bis zu 15 % Befall, während die unbehandelten Pflanzen zu 85 % befallen waren.

## Patentansprüche

1. 7-Aminotriazolopyrimidine der Formel I, 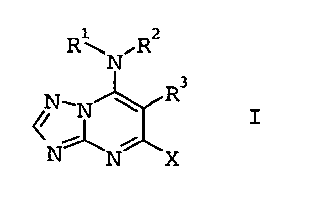 in der die Substituenten die folgenden Bedeutungen haben:
R¹, R² Wasserstoff, C₁-C₁₀-Alkyl, C₂-C₁₀-Alkenyl, C₂-C₁₀-Alkinyl, C₃-C₈-Cycloalkyl, Phenyl, Naphthyl; oder
5- oder 6-gliedriges Heterocyclyl enthaltend ein bis vier Stickstoffatome oder ein bis drei Stickstoffatome und ein Schwefel- oder Sauerstoffatom; oder
5- oder 6-gliedriges Heteroaryl enthaltend ein bis vier Stickstoffatome oder ein bis drei Stickstoffatome und ein Schwefel- oder Sauerstoffatom,
wobei R¹ und R², wenn ungleich Wasserstoff, unabhängig voneinander teilweise oder vollständig halogeniert sein können und/oder ein bis drei Reste aus der Gruppe R^{a}
R^{a} Cyano, Nitro, Hydroxy, C₁-C₆-Alkyl, C₁-C₆-Haloalkyl, C₃-C₆-Cycloalkyl, C₁-C₆-Alkoxy, C₁-C₆-Haloalkoxy, C₁-C₆-Alkylthio, C₁-C₆-Alkylamino, Di-C₁-C₆-alkylamino, C₂-C₆-Alkenyl, C₂-C₆-Alkenyloxy, C₂-C₆-Alkinyl, C₃-C₆-Alkinyloxy und gegebenenfalls halogeniertes Oxy-C₁-C₄-alkylenoxy,
tragen können; oder
R¹ und R² können zusammen mit dem Stickstoffatom, das sie verbindet, einen 5- oder 6-gliedrigen Ring bilden, der ein bis vier Stickstoffatome oder ein bis drei Stickstoffatome und ein Schwefel- oder Sauerstoffatom enthält, und der durch ein bis drei Reste aus der Gruppe R^{a} substituiert sein kann;
R³ C₁-C₁₀-Alkyl, C₂-C₁₀-Alkenyl, C₂-C₁₀-Alkinyl-, C₃-C₈-Cycloalkyl, Phenyl-C₁-C₁₀-Alkyl,
wobei R³ unsubstituiert oder teilweise oder vollständig halogeniert sein kann und/oder ein bis drei Reste aus der Gruppe R^{a} tragen kann, oder
C₁-C₁₀-Haloalkyl, das ein bis drei Reste aus der Gruppe R^{a} tragen kann;
X Halogen, Cyano, C₁-C₄-Alkoxy, C₁-C₄-Haloalkyl, Phenyl oder durch R^{a} substituiertes Phenyl;
sowie deren Salze.

2. 7-Aminotriazolopyrimidine der Formel I gemäß Anspruch 1, in der X für Halogen steht.

3. Verfahren zur Herstellung von 7-Aminotriazolopyrimidinen der Formel I nach Anspruch 1, in der X Halogen, Cyano oder C₁-C₄-Alkoxy bedeutet, **dadurch gekennzeichnet, daß** man Dicarbonyl-verbindungen der Formel II. 1, 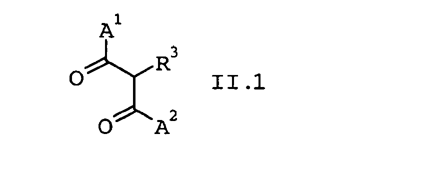 wobei A¹ und A² für C₁-C₁₀-Alkoxy stehen, mit
3-Amino-1,2,4-triazol der Formel III 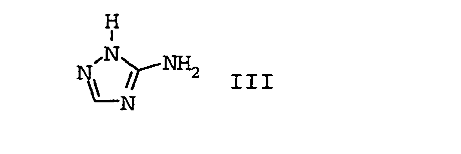 zu Hydroxytriazolopyrimidinen der Formel IV.1 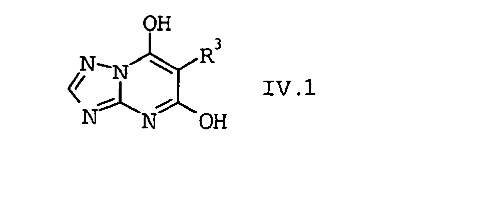 cyclisiert, die Hydroxytriazolopyrimidine der Formel IV.1 mit einem Halogenierungsmittel zu Halogentriazolopyrimidinen der Formel V.1 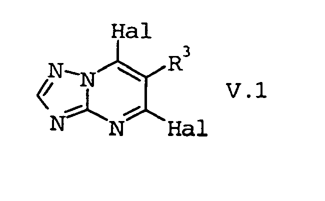 halogeniert, wobei Hal für Halogen steht, und anschließend mit einem Amin der Formel VI 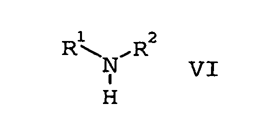 zu 7-Aminotriazolopyrimidinen der Formel I , in denen X Halogen bedeutet, umsetzt, und zur Herstellung von 7-Aminotriazolopyrimidinen der Formel I, in denen X Cyano oder C₁-C₄-Alkoxy bedeutet, mit einer Verbindung der Formel VII
M-X' VII
in der M ein Ammonium-, Tetraalkylammonium-, Alkalimetall- oder Erdalkalimetallkation und X' Cyano oder Alkoxy bedeutet, umsetzt.

4. (5-Chlor-6-ethyl-[1,2,4]triazolo[1,5-a]pyrimidin-7-yl)-cyclopentyl-amin;
(5-Chlor-6-ethyl-[1,2,4]triazolo[1,5-a]pyrimidin-7-yl)-(S)-(1,2-dimethyl-propyl)-amin;
(5-Chlor-6-ethyl-[1,2,4]triazolo[1,5-a]pyrimidin-7-yl)-(*S*)-(2,2,2-trifluor-1-methylethyl)-amin;
6-Butyl-5-chlor-7-(4-methyl-piperidin-1-yl)-[1,2,4]triazolo[1,5-a]pyrimidin;
(5-Chlor-6-octyl-[1,2,4]triazolo[1,5-a]pyrimidin-7-yl)-dipropyl-amin;
(5-Chlor-6-isopropyl-[1,2,4]triazolo[1,5-a]pyrimidin-7-yl)-ethyl-(2-methyl-allyl)-amin;
(5-Chlor-6-isopropyl-[1,2,4]triazolo[1,5-a]pyrimidin-7-yl)-isopropyl-amin;
(5-Chlor-6-isopropyl-[1,2,4]triazolo[1,5-a]pyrimidin-7-yl)-diethyl-amin;
(*R*/*S*)-sec-Butyl-(5-chlor-6-isopropyl-[1,2,4]triazolo[1,5-a]pyrimidin-7-yl)-amin;
(*R*)-sec-Butyl-(5-chlor-6-isopropyl-[1,2,4]triazolo[1,5-a]pyrimidin-7-yl)-amin;
(5-Chlor-6-isopropyl-[1,2,4]triazolo[1,5-a]pyrimidin-7-yl)-(*R*/*S*)-(1,2-dimethyl-propyl)-amin;
(5-Chlor-6-isopropyl-[1,2,4]triazolo[1,5-a]pyrimidin-7-yl)-(*R*)-(1,2-dimethyl-propyl)-amin;
(5-Chlor-6-isopropyl-[1,2,4]triazolo[1,5-a]pyrimidin-7-yl)-(*S*)-(2,2,2-trifluor-1-methyl-ethyl)-amin;
(5-Chlor-6-cyclopentyl-[1,2,4]triazolo[1,5-a]pyrimidin-7-yl)-ethyl-(2-methyl-allyl)-amin;
(5-Chlor-6-cyclohexyl-[1,2,4]triazolo[1,5-a]pyrimidin-7-yl)-(*S*)-(2,2,2-trifluor-1-methyl-ethyl)-amin;
(5-Chlor-6-cyclohexyl-[1,2,4]triazolo[1,5-a]pyrimidin-7-yl)-ethyl-amin;
[5-Chlor-6-(2,2,2-trifluor-ethyl)-[1,2,4]triazolo[1,5-a]pyrimidin-7-yl]-cyclopentyl-amin;
6-Octyl-5-trifluormethyl-[1,2,4]triazolo[1,5-a]pyrimidin-7-ylamin;
sec-Butyl-(5-chlor-6-cyclopentyl-[1,2,4]triazolo[1,5-a]pyrimidin-7-yl)-amin;
(5-Chlor-6-cyclopentyl-[1,2,4]triazolo[1,5-a]pyrimidin-7-yl)-(*R*)-(1,2,2-trimethyl-propyl)-amin;
(5-Chlor-6-cyclopentyl-[1,2,4]triazolo[1,5-a]pyrimidin-7-yl)-*(R*/*S)*-(2,2,2-trifluor-1-methyl-ethyl)-amin;
(5-Chlor-6-cyclopentyl-[1,2,4]triazolo[1,5-a]pyrimidin-7-yl)-(*S*)-(2,2,2-trifluor-1-methyl-ethyl)-amin.

5. (5-Chlor-6-isobutyl-[1,2,4]triazolo[1,5-a]pyrimidin-7-yl)-(2-methyl-allyl)-amin;
(5-Chlor-6-isobutyl-[1,2,4]triazolo[1,5-a]pyrimidin-7-yl)-isopropyl-amin;
sec-Butyl-(5-chlor-6-isobutyl-[1,2,4]triazolo[1,5-a]pyrimidin-7-yl)-amin;
(5-Chlor-6-isopropyl-[1,2,4]triazolo[1,5-a]pyrimidin-7-yl)-isopropyl-amin;
5-Chlor-6-cyclohexyl-7-(4-methyl-piperidin-1-yl)-[1,2,4]triazolo[1,5-a]pyrimidin;
(5-Chlor-6-cyclopentyl-[1,2,4]triazolo[1,5-a]pyrimidin-7-yl)-cyclopentyl-amin;
(5-Chlor-6-cyclopentyl-[1,2,4]triazolo[1,5-a]pyrimidin 7-yl)-(*R*/*S*)-(1,2,2-trimethyl-propyl)-amin.

6. 6-Isobutyl-5-trifluormethyl-[1,2,4]triazolo[1,5-a]pyrimidin-7-ylamin ;
6-(1-Methyl-butyl)-5-trifluormethyl-[1,2,4]triazolo[1,5-a]pyrimidin-7-ylamin;
6-tert-Butyl-5-trifluormethyl-[1,2,4]triazolo[1,5-a]pyrimidin-7-ylamin;
6-Isopropyl-5-trifluormethyl-[1,2,4]triazolo[1,5-a]pyrimidin-7-ylamin;
6-Cyclopentyl-5-trifluormethyl-[1,2,4]triazolo[1,5-a]pyrimidin-7-ylamin;
6-Cyclohexyl-5-trifluormethyl-[1,2,4]triazolo[1,5-a]pyrimidin-7-ylamin;
6-Benzyl-5-trifluormethyl-[1,2,4]triazolo[1,5-a]pyrimidin-7-ylamin;
6-(4-Chlor-benzyl)-5-trifluormethyl-[1,2,4]triazolo[1,5-a]pyrimidin-7-ylamin;
5-Chlor-6-ethyl-[1,2,4]triazolo[1,5-a]pyrimidin-7-ylamin;
5-Chlor-6-isopropyl-[1,2,4]triazolo[1,5-a]pyrimidin-7-ylamin;
5-Chlor-6-(2,2,2-trifluor-ethyl)-[1,2,4]triazolo[1,5-a]pyrimidin-7-ylamin;
6-Propyl-5-trifluormethyl-[1,2,4]triazolo[1,5-a]pyrimidin-7-ylamin;
6-(1-Methyl-heptyl)-5-trifluormethyl-[1,2,4]triazolo[1,5-a]pyrimidin-7-ylamin ;
6-(4-Methyl-pentyl)-5-trifluormethyl-[1,2,4]triazolo[1,5-a]pyrimidin-7-ylamin;
6-Heptyl-5-trifluormethyl-[1,2,4]triazolo[1,5-a]pyrimidin-7-ylamin ;
6-Hexyl-5-trifluormethyl-[1,2,4]triazolo[1,5-a]pyrimidin-7-ylamin ;
6-(1-Ethyl-pentyl)-5-trifluormethyl-[1,2,4]triazolo[1,5-a]pyrimidin-7-ylamin;
6-(1-Propyl-butyl)-5-trifluormethyl-[1,2,4]triazolo[1,5-a]pyrimidin-7-ylamin;
6-(1-Methyl-pentyl)-5-trifluormethyl-[1,2,4]triazolo[1,5-a]pyrimidin-7-ylamin;
5-Chlor-6-octyl-[1,2,4]triazolo[1,5-a]pyrimidin-7-ylamin;
5-Chlor-6-cyclopentyl-[1,2,4]triazolo[1,5-a]pyrimidin-7-ylamin;
5-Chlor-6-cyclohexyl-[1,2,4]triazolo[1,5-a]pyrimidin-7-ylamin.

7. Verfahren zur Herstellung von Verbindungen der Formel I nach Anspruch 1, in der X C₁-C₄-Haloalkyl oder gegebenenfalls durch R^{a} substituiertes Phenyl bedeutet, **dadurch gekennzeichnet, daß** man Dicarbonylverbindungen der Formel II.2 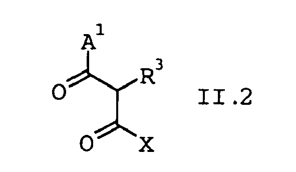 wobei A¹ für C₁-C₁₀-Alkoxy und X für C₁-C₄-Haloalkyl oder gegebenenfalls durch R^{a} substituiertes Phenyl steht, mit 3-Amino-1,2,4-triazol der Formel III nach Anspruch 3 zu 7-Hydroxytriazolopyrimidinen der Formel IV.2 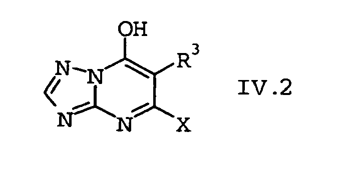 cyclisiert, die 7-Hydroxytriazolopyrimidine der Formel IV.2 mit einem Halogenierungsmittel zu 7-Halogentriazolopyrimidinen der Formel V.2 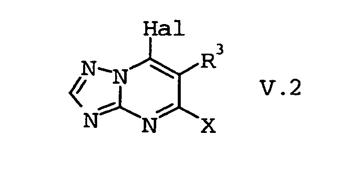 halogeniert, wobei Hal für Halogen steht, und anschließend mit einem Amin der Formel VI gemäß Anspruch 3 zu 7-Aminotriazolo-pyrimidinen der Formel I umsetzt.

8. Zur Bekämpfung von Schadpilzen geeignetes Mittel, enthaltend einen festen oder flüssigen Trägerstoff und ein 7-Aminotriazolo-pyrimidin der allgemeinen Formel I gemäß Anspruch 1.

9. Verwendung der 7-Aminotriazolopyrimidine der allgemeinen Formel I gemäß Anspruch 1 zur Herstellung eines zur Bekämpfung von Schadpilzen geeigneten Mittels.

10. Verfahren zur Bekämpfung von Schadpilzen, **dadurch gekennzeichnet, daß** man die Pilze oder die vor Pilzbefall zu schützenden Materialien, Pflanzen, den Boden oder Saatgüter mit einer wirksamen Menge der 7-Aminotriazolopyrimidine der allgemeinen Formel I gemäß Anspruch 1 behandelt.

## Claims

1. A 7-aminotriazolopyrimidine of the formula I, 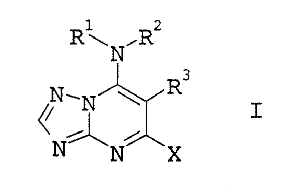 where:
R¹, R² are hydrogen, C₁-C₁₀-alkyl, C₂-C₁₀-alkenyl, C₂-C₁₀-alkynyl, C₃-C₈-cycloalkyl, phenyl, naphthyl; or
5- or 6-membered heterocyclyl containing one to four nitrogen atoms or one to three nitrogen atoms and one sulfur or oxygen atom; or
5- or 6-membered heteroaryl containing one to four nitrogen atoms or one to three nitrogen atoms and one sulfur or oxygen atom,
where R¹ and R², independently of one another, may, if they are not hydrogen, be partially or fully halogenated and/or may carry one to three radicals from the group R^{a}
R^{a} is cyano, nitro, hydroxyl, C₁-C₆-alkyl, C₁-C₆-haloalkyl, C₃-C₆-cycloalkyl, C₁-C₆-alkoxy, C₁-C₆-haloalkoxy, C₁-C₆-alkylthio, C₁-C₆-alkylamino, di-C₁-C₆-alkylamino, C₂-C₆-alkenyl, C₂-C₆-alkenyloxy, C₂-C₆-alkynyl, C₃-C₆-alkynyloxy and unhalogenated or halogenated oxy-C₁-C₄-alkyleneoxy;
or
R¹ and R² together with the linking nitrogen atom may form a 5- or 6-membered ring which contains one to four nitrogen atoms or one to three nitrogen atoms and one sulfur or oxygen atom and which may be substituted by one to three radicals from the group R^{a};
R³ is C₁-C₁₀-alkyl, C₂-C₁₀-alkenyl, C₂-C₁₀-alkynyl-, C₃-C₈-cycloalkyl, phenyl-C₁-C₁₀-alkyl,
where R³ may be unsubstituted or partially or fully halogenated and/or may carry one to three radicals from the group R^{a}, or
C₁-C₁₀-haloalkyl which may carry one to three radicals from the group R^{a};
X is halogen, cyano, C₁-C₄-alkoxy, C₁-C₄-haloalkyl, phenyl or R^{a}-substituted phenyl;
and its salts.

2. The 7-aminotriazolopyrimidine of the formula I according to claim 1 in which X is halogen.

3. A process for preparing 7-aminotriazolopyrimidines of the formula I according to claim 1 in which X is halogen, cyano or C₁-C₄-alkoxy, which comprises cyclizing dicarbonyl compounds of the formula II.1, 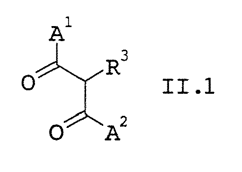 where A¹ and A² are C₁-C₁₀-alkoxy, with 3-amino-1,2,4-triazole of the formula III 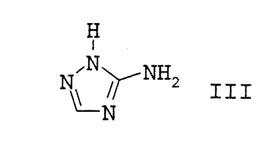 to give hydroxytriazolopyrimidines of the formula IV.1 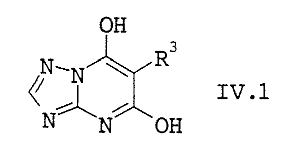 halogenating the hydroxytriazolopyrimidines of the formula IV.1 with a halogenating agent to give halotriazolopyrimidines of the formula V.1 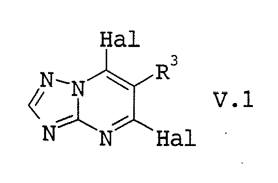 where Hal is halogen, followed by reaction with an amine of the formula VI 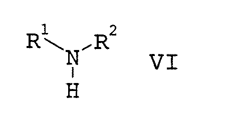 to give 7-aminotriazolopyrimidines of the formula I in which X is halogen, and, to prepare 7-aminotriazolopyrimidines of the formula I in which X is cyano or C₁-C₄-alkoxy, reacting with a compound of the formula VII
M-X' VII
in which M is an ammonium, tetraalkylammonium, alkali metal or alkaline earth metal cation and X' is cyano or alkoxy.

4. (5-Chloro-6-ethyl[1,2,4]triazolo[1,5-a]pyrimidin-7-yl)-cyclopentylamine;
(5-chloro-6-ethyl[1,2,4]triazolo[1,5-a]pyrimidin-7-yl)-(*S*)-(1,2-dimethylpropyl)amine;
(5-chloro-6-ethyl[1,2,4]triazolo[1,5-a]pyrimidin-7-yl)-(*S*)-(2,2,2-trifluoro-1-methylethyl)amine;
6-butyl-5-chloro-7-(4-methylpiperidin-1-yl)[1,2,4]triazolo-[1,5-a]pyrimidine;
(5-chloro-6-octyl[1,2,4]triazolo[1,5-a]pyrimidin-7-yl)-dipropylamine;
(5-chloro-6-isopropyl[1,2,4]triazolo[1,5-a]pyrimidin-7-yl)-ethyl-(2-methylallyl)amine;
(5-chloro-6-isopropyl[1,2,4]triazolo[1,5-a]pyrimidin-7-yl)-isopropylamine;
(5-chloro-6-isopropyl[1,2,4]triazolo[1,5-a]pyrimidin-7-yl)-diethylamine;
(*R*/*S*)-sec-butyl-(5-chloro-6-isopropyl[1,2,4]triazolo[1,5-a]-pyrimidin-7-yl)amine;
(*R*)-sec-butyl-(5-chloro-6-isopropyl[1,2,4]triazolo[1,5-a]-pyrimidin-7-yl)amine;
(5-chloro-6-isopropyl[1,2,4]triazolo[1,5-a]pyrimidin-7-yl)-(*R*/*S*)-(1,2-dimethylpropyl)amine;
(5-chloro-6-isopropyl[1,2,4]triazolo[1,5-a]pyrimidin-7-yl)-(*R*)-(1,2-dimethylpropyl)amine;
(5-chloro-6-isopropyl[1,2,4]triazolo[1,5-a]pyrimidin-7-yl)-(*S*)-(2,2,2-trifluoro-1-methylethyl)amine;
(5-chloro-6-cyclopentyl[1,2,4]triazolo[1,5-a]pyrimidin-7-yl)ethyl-(2-methylallyl)amine;
(5-chloro-6-cyclohexyl[1,2,4]triazolo[1,5-a]pyrimidin-7-yl)-(*S*)-(2,2,2-trifluoro-1-methylethyl)amine;
(5-chloro-6-cyclohexyyl[1,2,4]triazolo[1,5-a]pyrimidin-7-yl)ethylamine;
(5-chloro-6-(2,2,2-trifluoroethyl)[1,2,4]triazolo[1,5-a]-pyrimidin-7-yl)cyclopentylamine;
6-octyl-5-trifluoromethyl[1,2,4]triazolo[1,5-a]pyrimidin-7-ylamine;
sec-butyl-(5-chloro-6-cyclopentyl[1,2,4]triazolo[1,5-a]-pyrimidin-7-yl)amine;
(5-chloro-6-cyclopentyl[1,2,4]triazolo[1,5-a]pyrimidin-7-yl)-(*R*)-(1,2,2-trimethylpropyl)amine;
(5-chloro-6-cyclopentyl[1,2,4]triazolo[1,5-a]pyrimidin-7-yl)-(*R*/*S*)-(2,2,2-trifluoro-1-methylethyl)amine;
(5-chloro-6-cyclopentyl[1,2,4]triazolo[1,5-a]pyrimidin-7-yl)-(*S*)-(2,2,2-trifluoro-l-methylethyl)amine.

5. (5-Chloro-6-isobutyl[1,2,4]triazolo[1,5-a]pyrimidin-7-yl)-(2-methylallyl)amine;
(5-chloro-6-isobutyl[1,2,4]triazolo[1,5-a]pyrimidin-7-yl)-isopropylamine;
sec-butyl-(5-chloro-6-isobutyl[1,2,4]triazolo[1,5-a]-pyrimidin-7-yl)amine;
(5-chloro-6-isopropyl[1,2,4]triazolo[1,5-a]pyrimidin-7-yl)-isopropylamine;
(5-chloro-6-cyclohexyl-7-(4-methylpiperidin-1-yl)[1,2,4]-triazolo[1,5-a]pyrimidine;
(5-chloro-6-cyclopentyl[1,2,4]triazolo[1,5-a]pyrimidin-7-yl)cyclopentylamine;
(5-chloro-6-cyclopentyl[1,2,4]triazolo[1,5-a]pyrimidin-7-yl)-(*R*/*S*)-(1,2,2-trimethylpropyl)amine.

6. 6-Isobutyl-5-trifluoromethyl[1,2,4]triazolo[1,5-a]-pyrimidin-7-ylamine;
6-(1-methylbutyl)-5-trifluoromethyl[1,2,4]triazolo[1,5-a]-pyrimidin-7-ylamine;
6-tert-butyl-5-trifluoromethyl[1,2,4]triazolo[1,5-a]-pyrimidin-7-ylamine;
6-isopropyl-5-trifluoromethyl[1,2,4]triazolo[1,5-a]-pyrimidin-7-ylamine;
6-cyclopentyl-5-trifluoromethyl[1,2,4]triazolo[1,5-a]-pyrimidin-7-ylamine;
6-cyclohexyl-5-trifluoromethyl[1,2,4]triazolo[1,5-a]-pyrimidin-7-ylamine;
6-benzyl-5-trifluoromethyl[1,2,4]triazolo[1,5-a]pyrimidin-7-ylamine;
6-(4-chlorobenzyl)-5-trifluoromethyl[1,2,4]triazolo[1,5-a]-pyrimidin-7-ylamine;
5-chloro-6-ethyl[1,2,4]triazolo[1,5-a]pyrimidin-7-ylamine ;
5-chloro-6-isopropyl[1,2,4]triazolo[1,5-a]pyrimidin-7-yl-amine;
5-chloro-6-(2,2,2-trifluoroethyl)[1,2,4]triazolo[1,5-a]-pyrimidin-7-ylamine;
6-propyl-5-trifluoromethyl[1,2,4]triazolo[1,5-a]pyrimidin-7-ylamine;
6-(1-methylheptyl)-5-trifluoromethyl[1,2,4]triazolo[1,5-a]-pyrimidin-7-ylamine;
6-(4-methylpentyl)-5-trifluoromethyl[1,2,4]triazolo[1,5-a]-pyrimidin-7-ylamine;
6-heptyl-5-trifluoromethyl[1,2,4]triazolo-[1,5-a]pyrimidin-7-ylamine;
6-hexyl-5-trifluoromethyl[1,2,4]triazolo[1,5-a]pyrimidin-7-ylamine;
6-(1-ethylpentyl)-5-trifluoromethyl[1,2,4]triazolo[1,5-a]-pyrimidin-7-ylamine;
6-(1-propylbutyl)-5-trifluoromethyl[1,2,4]triazolo[1,5-a]-pyrimidin-7-ylamine;
6-(1-methylpentyl)-5-trifluoromethyl[1,2,4]triazolo[1,5-a]-pyrimidin-7-ylamine;
5-chloro-6-octyl[1,2,4]triazolo[1,5-a]pyrimidin-7-ylamine;
5-chloro-6-cyclopentyl[1,2,4]triazolo[1,5-a]pyrimidin-7-ylamine;
5-chloro-6-cyclohexyl[1,2,4]triazolo[1,5-a]pyrimidin-7-yl-amine.

7. A process for preparing compounds of the formula I according to claim 1, in which X is C₁-C₄-haloalkyl or unsubstituted or R^{a}-substituted phenyl, which comprises cyclizing dicarbonyl compounds of the formula II.2 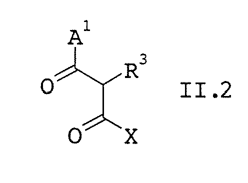 where A¹ is C₁-C₁₀-alkoxy and X is C₁-C₄-haloalkyl or unsubstituted or R^{a}-substituted phenyl with 3-amino-1,2,4-triazole of the formula III according to claim 3 to give 7-hydroxytriazolopyrimidines of the formula IV.2 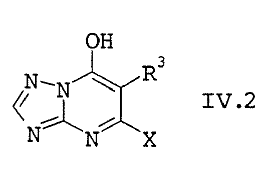 halogenating the 7-hydroxytriazolopyrimidines of the formula IV.2 with a halogenating agent to give 7-halotriazolopyrimidines of the formula V.2 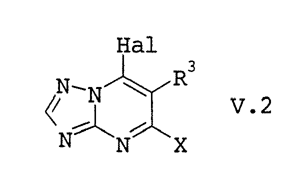 where Hal is halogen, followed by reaction with an amine of the formula VI according to claim 3 to give 7-aminotriazolopyrimidines of the formula I.

8. A composition suitable for controlling harmful fungi, which comprises a solid or liquid carrier and a 7-aminotriazolopyrimidine of the formula I according to claim 1.

9. The use of the 7-aminotriazolopyrimidines of the formula I according to claim 1 for preparing a composition suitable for controlling harmful fungi.

10. A method for controlling harmful fungi, which comprises treating the fungi or the materials, plants, the soil or the seeds to be protected against fungal attack with an effective amount of the 7-aminotriazolopyrimidines of the formula I according to claim 1.

## Revendications

1. 7-Aminotriazolopyrimidines de formule I : 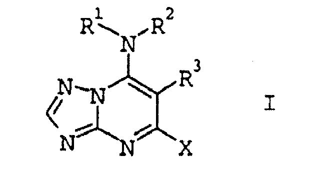 dans laquelle les substituants ont les significations suivantes :
R¹, R² représentent de l'hydrogène ou un groupe alkyle en C₁-C₁₀, alcényle en C₂-C₁₀, alcynyle en C₂-C₁₀, cycloalkyle en C₃-C₈, phényle ou naphtyle, ou
un groupe hétérocyclyle à 5 ou 6 termes contenant un à quatre atomes d'azote ou un à trois atomes d'azote et un atome de soufre ou d'oxygène,
un groupe hétéroaryle à 5 ou 6 termes contenant un à quatre atomes d'azote ou un à trois atomes d'azote et un atome de soufre ou d'oxygène,
R¹ et R² pouvant, lorsqu'ils sont différents de l'hydrogène, être indépendamment l'un de l'autre partiellement ou totalement halogénés et/ou pouvant porter un à trois radicaux du groupe R^{a},
R^{a} étant un groupe cyano, nitro, hydroxy, alkyle en C₁-C₆, halogénoalkyle en C₁-C₆, cycloalkyle en C₃-C₆, alcoxy en C₁-C₆, halogénoalcoxy en C₁-C₆, alkylthio en C₁-C₆, alkylamino en C₁-C₆, di-C₁-C₆-alkylamino, alcényle en C₂-C₆, alcényloxy en C₂-C₆, alcynyle en C₂-C₆, alcynyloxy en C₃-C₆ et oxy-C₁-C₄-alkylènoxy éventuellement halogéné,
ou
R¹ et R² pouvant former conjointement avec l'atome d'azote qui les relie un noyau pentagonal ou hexagonal qui contient un à quatre atomes d'azote ou un à trois atomes d'azote et un atome de soufre ou d'oxygène et qui peut être substitué par un à trois radicaux du groupe R^{a},
R³ représente un groupe alkyle en C₁-C₁₀, alcényle en C₂-C₁₀, alcynyle en C₂-C₁₀, cycloalkyle en C₃-C₈, phényl-C₁-C₁₀-alkyle,
R³ pouvant être non substitué ou partiellement ou totalement halogéné et/ou pouvant porter un à trois radicaux du groupe R^{a}, ou
un groupe halogénoalkyle en C₁-C₁₀ qui peut porter un à trois radicaux du groupe R^{a},
X représente de l'halogène ou un groupe cyano, alcoxy en C₁-C₄, halogénoalkyle en C₁-C₄, phényle ou phényle substitué par R^{a},
ainsi que leurs sels.

2. 7-Aminotriazolopyrimidines de formule I suivant la revendication 1, dans lesquelles X représente de l'halogène.

3. Procédé de préparation de 7-aminotriazolopyrimidines de formule I suivant la revendication 1, dans lesquelles X représente de l'halogène ou un groupe cyano ou alcoxy en C₁-C₄, **caractérisé en ce qu'**on cyclise des composés dicarbonylés de formule II.1 : 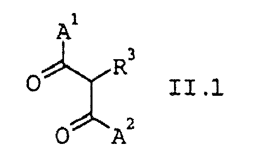 où A¹ et A² représentent un groupe alcoxy en C₁-C₁₀, avec du 3-amino-1,2,4-triazole de formule III : 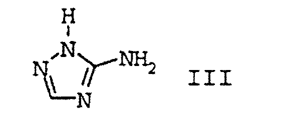 pour former des hydroxytriazolopyrimidines de formule IV.1: 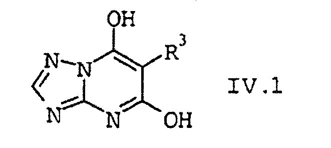 on halogène les hydroxytriazolopyrimidines de formule IV.1 avec un agent d'halogénation pour former des halogénotriazolopyrimidines de formule V.1 : 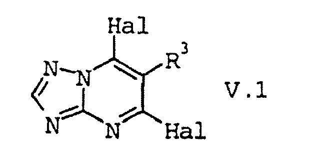 Hal représentant de l'halogène, et ensuite on fait réagir avec une amine de formule VI : 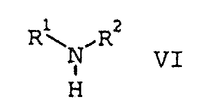 pour former des 7-aminotriazolopyrimidines de formule I dans lesquelles X représente de l'halogène, et, pour la préparation de 7-aminotriazolopyrimidines de formule I dans lesquelles X représente un groupe cyano ou alcoxy en C₁-C₄, on fait réagir avec un composé de formule VII:
M- X' VII
dans laquelle M représente un cation ammonium, tétraalkylammonium, de métal alcalin ou de métal alcalino-terreux et X' représente un groupe cyano ou alcoxy.

4. (5-Chloro-6-éthyl[1,2,4]triazolo[1,5-a]pyrimidin-7-yl)cyclopentylamine;
(5-chloro-6-éthyl[1,2,4]triazolo[1,5-a]pyrimidin-7-yl)-(*S*)-(1,2-diméthylpropyl)amine;
(5-chloro-6-éthyl[1,2,4]triazolo[1,5-a]pyrimidin-7-yl)-(*S*)-(2,2,2-trifluoro-1-méthyléthyl)amine;
6-butyl-5-chloro-7-(4-méthylpipéridin-1-yl)-[1,2,4]triazolo[1,5-a]pyrimidine;
(5-chloro-6-octyl[1,2,4]triazolo[1,5-a]pyrimidin-7-yl)dipropylamine;
(5-chloro-6-isopropyl[1,2,4]triazolo[1,5-a]pyrimidin-7-yl)éthyl-(2-méthylallyl)amine;
(5-chloro-6-isopropyl[1,2,4]triazolo[1,5-a]pyrimidin-7-yl)isopropylamine;
(5-chloro-6-isopropyl[1,2,4]triazolo[1,5-a]pyrimidin-7-yl)diéthylamine;
(*R*/*S*)-sec-butyl-(5-chloro-6-isopropyl-[1,2,4]triazolo[1,5-a]pyrimidin-7-yl)aminé;
(*R*)-sec-butyl-(5-chloro-6-isopropyl-[1,2,4] triazolo[1,5-a]pyrimidin-7-yl)amine;
(5-chloro-6-isopropyl[1,2,4]triazolo[1,5-a]pyrimidin-7-yl)-(*R*/*S*)-(1,2-diméthylpropyl)-amine;
(5-chloro-6-isopropyl[1,2,4]triazolo[1,5-a]pyrimidin-7-yl)-(*R*)-(1,2-diméthylpropyl)amine;
(5-chloro-6-isopropyl[1,2,4]triazolo[1,5-a]pyrimidin-7-yl)-(*S*)-(2,2,2-trifluoro-1-méthyl-éthyl)amine;
(5-chloro-6-cyclopentyl-[1,2,4]triazolo[1,5-a]pyrimidin-7-yl)éthyl-(2-méthylallyl)amine;
(5-chloro-6-cyclohexyl-[1,2,4]triazolo[1,5-a]pyrimidin-7-yl)-(*S*)-(2,2,2-trifluoro-1-méthyl-éthyl)amine;
(5-chloro-6-cyclohexyl-[1,2,4]triazolo[1,5-a]pyrimidin-7-yl)éthyl)amine;
[5-chloro-6-(2,2,2-trifluoro-éthyl)-[1,2,4] triazolo[1,5-a]pyrimidin-7-yl]cyclopentyl-amine;
6-octyl-5-trifluorométhyl[1,2,4]triazolo[1,5-a]pyrimidin-7-ylamine;
sec-butyl-(5-chloro-6-cyclopentyl-[1,2,4]triazolo[1,5-a]pyrimidin-7-yl)aminé;
(5-chloro-6-cyclopentyl[1,2,4]triazolo[1,5-a]pyrimidin-7-yl)-(*R*)-(1,2,2-triméthylpropyl)-amine;
(5-chloro-6-cyclopentyl[1,2,4]triazolo[1,5-a]pyrimidin-7-yl)-(*R*/*S*)-(2,2,2-trifluoro-1-méthyl-éthyl)amine;
(5-chloro-6-cyclopentyl[1,2,4]triazolo[1,5-a]pyrimidin-7-yl)-(*S*)-(2,2,2-trifluoro-1-méthyl-éthyl)amine.

5. (5-Chloro-6-isobutyl[1,2,4]triazolo[1,5-a]pyrimidin-7-yl)-(2-méthylallyl)amine;
(5-chloro-6-isobutyl[1,2,4]triazolo[1,5-a]pyrimidin-7-yl}isopropylamine;
sec-butyl-(5-chloro-6-isobutyl[1,2,4]triazolo[1,5-a] pyrimidin-7-yl)amine ;
(5-chloro-6-isopropyl[1,2,4]triazolo[1,5-a]pyrimidin-7-yl)isopropylamine;
(5-chloro-6-cyclohexyl-7-(4-méthylpipéridin-1-yl)-[1,2,4]triazolo [1,5-a] pyrimidine;
(5-chloro-6-cyclopentyl[1,2,4]triazolo[1,5-a]pyrimidin-7-yl)cyclopentylamine;
(5-chloro-6-cyclopentyl[1,2,4]triazolo[1,5-a]pyrimidin-7-yl)-(*R*/*S*)-(1,2,2-triméthylpropyl)-amine.

6. 6-Isobutyl-5-trifluorométhyl[1,2,4]triazolo[1,5-a]pyrimidin-7-ylamine;
6-(1-méthylbutyl)-5-trifluorométhyl-[1,2,4]triazolo[1,5-a]pyrimidin-7-ylamine;
6-tert-butyl-5-trifluorométhyl[1,2,4]triazolo[1,5-a]pyrimidin-7-ylamine;
6-isopropyl-5-trifluorométhyl[1,2,4]triazolo[1,5-a]pyrimidin-7-ylamine;
6-cyclopentyl-5-trifluorométhyl-[1,2,4]triazolo[1,5-a]pyrimidin-7-ylamine;
6-cyclohexyl-5-trifluorométhyl[1,2,4]triazolo[1,5-a]pyrimidin-7-ylamine;
6-benzyl-5-trifluorométhyl[1,2,4]triazolo[1,5-a]pyrimidin-7-ylamine;
6-(4-chlorobenzyl)-5-trifluorométhyl-[1,2,4]triazolo[1,5-a]pyrimidin-7-ylamine;
5-chloro-6-éthyl[1,2,4]triazolo[1,5-a]pyrimidin-7-ylamine;
5-chloro-6-isopropyl[1,2,4]triazolo[1,5-a]pyrimidin-7-ylamine;
5-chloro-6-(2,2,2-trifluoroéthyl)-[1,2,4]triazolo[1,5-a]pyrimidin-7-ylamine;
6-propyl-5-trifluorométhyl[1,2,4]triazolo[1,5-a]pyrimidin-7-ylamine;
6-(1-méthylheptyl)-5-trifluorométhyl-[1,2,4]triazolo[1,5-a]pyrimidin-7-ylamine;
6-(4-méthylpentyl)-5-trifluorométhyl-[1,2,4]triazolo[1,5-a]pyrimidin-7-ylamine;
6-heptyl-5-trifluorométhyl[1,2,4]triazolo[1,5-a]pyrimidin-7-ylamine;
6-hexyl-5-trifluorométhyl[1,2,4]triazolo[1,5-a]pyrimidin-7-ylamine;
6-(1-éthylpentyl)-5-trifluorométhyl-[1,2,4]triazolo[1,5-a]pyrimidin-7-ylamine ;
6-(1-propylbutyl)-5-trifluorométhyl-[1, 2, 4] triazolo [1, 5-a] pyrimidin-7-ylamine;
6-(1-méthylpentyl)-5-trifluorométhyl-[1,2,4]triazolo[1,5-a]pyrimidin-7-ylamine;
5-chloro-6-octyl[1,2,4]triazolo[1,5-a]pyrimidin-7-ylamine;
5-chloro-6-cyclopentyl[1,2,4]triazolo[1,5-a]pyrimidin-7-ylamine;
5-chloro-6-cyclohexyl[1,2,4]triazolo[1,5-a]pyrimidin-7-ylamine.

7. Procédé de préparation de composés de formule I suivant la revendication 1, dans lesquels X représente un groupe halogénoalkyle en C₁-C₄ ou éventuellement est du phényle substitué par R^{a}, **caractérisé en ce qu'**on cyclise des composés dicarbonylés de formule II. 2 : 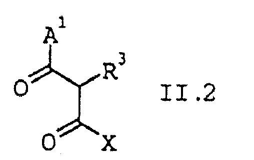 où A¹ représente un groupe alcoxy en C₁-C₁₀ et X un groupe halogénoalkyle en C₁-C₄ ou un groupe phényle éventuellement substitué par R^{a}, avec du 3-amino-1,2,4-triazole de formule III suivant la revendication 3 pour former des 7-hydroxytriazolopyrimidines de formule IV.2 : 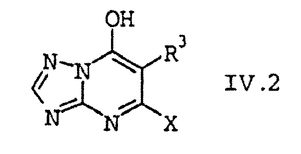 on halogène les 7-hydroxytriazolopyrimidines de formule IV.2 avec un agent d'halogénation pour former des 7-halogénotriazolopyrimidines de formule V.2 : 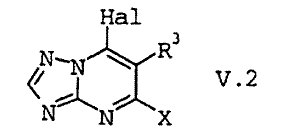 Hal représentant de l'halogène, et ensuite on fait réagir avec une amine de formule VI suivant la revendication 3 pour former des 7-aminotriazolopyrimidines de formule I.

8. Produit approprié pour lutter contre les champignons nuisibles, contenant un support solide ou liquide et une 7-aminotriazolopyrimidine de formule générale I suivant la revendication 1.

9. Utilisation des 7-aminotriazolopyrimidines de formule générale I suivant la revendication 1, pour la préparation d'un produit approprié pour lutter contre les champignons nuisibles.

10. Procédé pour lutter contre des champignons nuisibles, **caractérisé en ce qu'**on traite les champignons ou les matériaux, plantes, sols ou semences à protéger d'une infestation par des champignons par une quantité efficace de 7-aminotriazolopyrimidines de formule générale I suivant la revendication 1.
